# FASCICULE DE BREVET EUROPEEN

(11) **EP 1 387 837 B1**
(45) Date de publication et mention de la délivrance du brevet: **27.07.2005**
(21) Numéro de dépôt: 02730383.3
(22) Date de dépôt: 02.05.2002
(51) Int. Cl.: C07D 401/12, C07D 233/60, A61K 31/4725, A61K 31/4164, A61P 31/10

(54) **NOUVEAUX DERIVES D'AZOLE OU DE TRIAZOLE, LEUR PROCEDE DE PREPARATION ET LEUR APPLICATION COMME MEDICAMENTS ANTI-FONGIQUES**
AZOL- ODER TRIAZOL-DERIVATE, VERFAHREN ZU DEREN HERSTELLUNG UND IHRE VERWENDUNG ALS ANTIFUNGALE ARZNEIMITTEL
NOVEL AZOLE OR TRIAZOLE DERIVATIVES, METHOD FOR PREPARING SAME AND USE THEREOF AS ANTIFUNGAL MEDICINES

(30) Priorité: 04.05.2001 FR 0105959
(43) Date de publication de la demande: 11.02.2004
(73) Titulaire: Aventis Pharma S.A., 92160 Antony (FR)
(72) Inventeur: BABIN, Didier, F-78180 Montigny (FR); WESTON, John, 65779 Kelkheim (DE)
(74) Mandataire: Rousseau, Pierrick Edouard
(86) Numéro de dépôt international: PCT/FR2002/001519
(87) Numéro de publication internationale: WO 2002/090350

(56) Documents cités:
- EP-A- 0 050 298
- EP-A- 0 121 753
- WO-A-00/20413
- WO-A-01/74808

## Description

La présente invention concerne de nouveaux dérivés d'azole ou de triazole, leur procédé de préparation et leur application comme médicaments anti-fongiques.

De nombreux composés ayant une activité antifongique sont connus dans l'art antérieur. On peut citer notamment des dérivés d'azoles tels que définis dans les demandes suivantes : EP 0 121 753 A (Hoechst AG), EP 0 050 298 A (Hoechst AG), US 2,813,872 (J schmutz), WO 00/20413 (Hoechst Marion Roussel). Il existe néanmoins un réel besoin de mettre en oeuvre de nouveaux composés antifongiques, les souches actuelles pouvant être ou devenir résistantes aux agents conventionnels notamment lorsque ces derniers ne possèdent qu'une activité fongistatique. Par ailleurs, les nouveaux composés antifongiques doivent pouvoir présenter une solubilité améliorée et doivent pouvoir également être plus facilement absorbés. les Enfin, l'incidence du Candida Albicans comme agent infectieux, est de plus en plus importante, notamment vis-à-vis des patients immunodéprimés, par exemple suite à une infection au VIH, et nécessite donc de nouveaux traitements.

L'objectif de la présente invention est de fournir de nouveaux composés ayant une activité antifongique, notamment vis-à-vis des souches Candida ou Aspergillus.

L'invention a pour objet, les composés de formule (I) 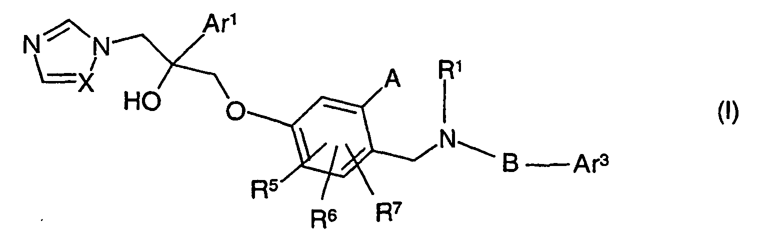 dans laquelle
- X est un atome d'azote ou un groupement CH,
- Ar¹ représente un aryle carbocyclique ou hétérocyclique non substitué ou substitué par un ou plusieurs radicaux R², R³ ou R⁴
- A représente un atome d'hydrogène ou représente un groupement CH₂ formant avec R1 une liaison carbone-carbone cyclique, afin d'obtenir un cycle à 6 chainons accolé au phényle,
- Ar³ représente un aryle carbocyclique ou hétérocyclique non substitué ou substitué par un ou plusieurs radicaux R⁸, R⁹ ou R¹⁰
- B représente un radical (C₁-C₄)-alkylène-CH=CH- ou un radical (C₁-C₄)-alkylène-cyclopropylène, les dits radicaux cyclopropylene ou -CH=CH- étant non substitués ou substitués par un radical R² et/ou R³,
- R¹ représente un atome d'hydrogène, un groupement -SO₃H, un radical (C₁-C₆)-alkyle non substitué ou substitué par un radical tel que défini pour R², ou représente un groupement CH₂ formant avec A une liaison carbone-carbone cyclique, afin d'obtenir un cycle à 6 chainons accolé au phényle,
- R², R³, R⁴, R⁵, R⁶, R⁷, R⁸, R⁹ ou R¹⁰ identiques ou différents, représentent fluor, chlore, brome, cyano, mono- bi- ou trihalogeno(C₁-C₈)alkyle, mono- bi- ou trihalogeno(C₁-C₈)-alkyloxy, hydroxy, nitro, carboxyle, formyle, -SO₃H, - OSO₃H, (R¹¹O)₂P(O)-, (R¹¹O)₂P(O)-O-, amino, (C₁-C₈)-alkyl-amino, di((C₁-C₈)alkyl)amino, (C₅-C₁₄) -aryl- (C₁-C₆)-alkylèn-amino ou (C₅-C₁₄)-arylamino, (C₁-C₈)-alkyle, (C₅-C₁₄)-aryle, un hétérocycle éventuellement substitué par oxo, (C₅-C₁₄)-aryl- (C₁-C₆)alkyle, amino-(C₁-C₆)-alkyle, (C₁-C₈)-alkylamino-(C₁-C₆)-alkyle, di- ((C₁-C₈)alkyl)amino-(C₁-C₆)-alkyle, hydroxy-(C₁-C₆)alkyle, (C₁-C₆)-alkyloxy- (C₁-C₆)-alkyle, (C₁-C₈)-alkyloxy éventuellement interrompu par un ou plusieurs atomes d'oxygène, (C₅-C₁₄)-aryl-(C₁-C₆)-alkylènoxy, (C₅-C₁₄)-aryloxy, hydroxy-(C₁-C₆)alkylènoxy, (C₁-C₆)-alkyloxy-(C₁-C₆)-alkylènoxy, amino-(C₁-C₆)-alkylènoxy, (C₁-C₆)-alkylamino-(C₁-C₆)-alkylènoxy, di((C₁-C₈)-alkyl)amino-(C₁-C₆)-alkylènoxy, méthylènedioxy, (C₁-C₆)-alkyloxycarbonyle, (C₁-C₆)-alkylcarbonyle, (C₅-C₁₄)aryl-(C₁-C₆)-alkylènecarbonyl, (C₅-C₁₄)-aryl-carbonyle, (C₁-C₆)-alkylaminocarbonyle, (C₁-C₆)-alkanoylamino, (C₁-C₆)-alkylsulfonylamino, (C₅-C₁₄)-aryl-sulfonylamino, (C₅-C₁₄)-aryl-(C₁-C₆)-alkylènesulfonylamino, (C₁-C₆)-alkylaminosulfonyle, (C₅-C₁₄)-aryl-(C₁-C₆)-alkylènaminosulfonyl, (C₁-C₆)-alkylsulfonyl, (C₅-C₁₄)-aryl-(C₁-C₈)-alkylènesulfonyle ou (C₅-C₁₄)-aryl-sulfonyle, les dits radicaux alkyles, aryles ou hétérocycles étant eux même non substitués ou substitués par un ou plusieurs groupements cités ci-dessus.
- R¹¹ représente hydrogène, (C₁-C₁₀)-alkyl, (C₆-C₁₄)-aryle ou (C₆-C₁₄)-aryl-(C₁-C₆)-alkyle,
   sous toutes leurs formes stéréoisomères possibles et leurs mélanges,ainsi que leurs sels d'addition physiologiquement acceptables et leurs prodrogues.

Tous les radicaux qui peuvent se retrouver plusieurs fois dans les composés de formule (I), par exemple, le radical R², sont indépendants les uns des autres et peuvent être identiques ou différents.

Les radicaux alkyles cités plus haut peuvent être linéaires, ramifiés ou cycliques (cycloalkyles). Ceci s'applique également lorsqu'ils portent un substituant ou lorsqu'ils sont inclus dans des groupements tels que par exemple alkoxy, alkoxycarbonyle ou aralkyle.

Par (C₁-C₈)-alkyle on entend les radicaux méthyle, éthyle, propyle, butyle, pentyle, hexyle, heptyle, octyle, nonyle, les n-isomères de ces radicaux, isopropyle, isobutyle, isopentyle, néopentyle, isohexyle, 3-méthyl-pentyle, 2,3,4-triméthylhexyle, sec-butyle, tert-butyle, tert-pentyle. Parmi les radicaux préférés on peut citer méthyle, éthyle, n-propyle, isopropyle, n-butyle, isobutyle, et tert-butyle. Par (C₁-C₆)-alkyle on entend les radicaux méthyle, éthyle, propyle, butyle, pentyle, hexyle et les n-isomères de ces radicaux.

Par radical alkyloxy interrompu par un ou plusieurs atomes d'oxygènes, on entend de préférence un radical du type O-CH₂-O-(CH₂)₂-O-CH₃.

Les radicaux alkylènes bivalents correspondant aux radicaux monovalents cités plus haut sont par exemple les radicaux méthylène, éthylène, 1,3-propylène, 1,2-propylène (=1-méthyléthylène), 2,3-butylène (=1,2-diméthyléthylène), 1,4-butylène, ou 1,6-hexylène.

Par radicaux alkylène bivalents insaturés on entend les radicaux alkénylène et alkynylène qui peuvent également être linéaires ou ramifiés. Il s'agit par exemple des radicaux vinylène, propénylène, éthynylène ou propynylène.

Les radicaux cycloalkyles peuvent être monocycliques, bicycliques ou tricycliques. Il s'agit par exemple des radicaux cyclopropyle, cyclobutyle, cyclopentyle, cyclohexyle, cycloheptyle, cyclooctyle, cyclononyle, cyclodécyle, cycloundécyle, cyclododécyle, cyclotétradécyle ou cyclooctadécyle qui le cas échéant peuvent être substitués par exemple par un alkyle renfermant de 1 à 4 atomes de carbone. Comme radicaux cycloalkyles substitués, on peut citer le 4 méthylcyclo-hexyle, le 2,3-diméthylcyclo-hexyle, le diméthylcyclopropyl et le dichlorocyclopropyle.

Sauf indication contraire, les radicaux alkyles ou cycloalkyles peuvent être non substitués ou substitués par un ou plusieurs radicaux identiques ou différents choisis parmi (C₁-C₆)-alkyle, (C₁-C₆)-alkoxy, hydroxyle, halogène tel que fluor, chlore et brome, nitro, amino, trifluorométhyle, mono, -OCF₃, cyano, carboxyl, -SO₃H, -OSO₃H, PO₃H₂, OPO₃H₂, (C₁-C₄)-alkoxycarbonyle, phényle, phénoxy, benzyle et benzyloxy. Bien entendu ceci s'applique également lorsque le radical alkyle fait parti d'un groupement le renfermant, par exemple tel que, (C₁-C₆)-alkyloxycarbonyle, (C₁-C₆)-alkylcarbonyle ou (C₁-C₆)-alkylaminocarbonyle

Par halogène on entend fluor, chlore, brome ou iode.

Par le terme aryle on entend :
- soit les radicaux (C₅-C₁₄)-aryle hétérocycliques (= (C₅-C₁₄)-hétéroaryle), dans lesquels les atomes de carbone du cycle sont remplacés par un hétéroatome tel que azote, oxygène ou soufre,
- soit les radicaux (C₆-C₁₄)-aryle carbocyclique.

Parmi les radicaux(C₆-C₁₄)-aryle carbocycliques, on peut citer le phényle, le naphtyle, le biphénylyle, l'anthryle ou le fluorényle et tout particulièrement le 1-naphtyle, le 2-naphtyle et le phényle.

Sauf indication contraire, les radicaux aryles, en particulier phényle, peuvent être non substitués ou substitués par un ou plusieurs radicaux identiques ou différents choisis parmi (C₁-C₆)-alkyle, (C₁-C₆)-alkoxy, hydroxyle, hydroxy(C₁-C₆)-alkyle, halogène tel que fluor, chlore et brome, nitro, amino, trifluorométhyle, -OCF₃, cyano, carboxyl, -SO₃H, -OSO₃H, PO₃H₂, OPO₃H₂, (C₁-C₄)-alkoxycarbonyle, phényle, phénoxy, benzyle, benzyloxy et méthylènedioxy.

Dans le cas du phényle monosubstitué, le substituant peut être situé en position 2, 3 ou 4, et de préférence en position 3 ou 4. De préférence, Ar³ représente un phényle substitué en position 4. Dans le cas où le phényle est disubstitué, les substituants peuvent être en position 2,3 ou 2,4 ou 2,5 ou 2,6 ou 3,4 ou 3,5. De préférence, lorsque Ar¹ représente un phényle disubstitué, les deux substituants sont en position 2,4. Lorsque ce phényle est tri-substitué les positions sont les suivants : 2,3,4 ou 2,3,5 ou 2,3,6 ou 2,4,5 ou 2,4,6 ou 3,4,5. De la même manière, les radicaux naphtyles ou d'autres radicaux aryles peuvent être substitués en toute position, par exemple le radical 1-naphtyle en position 2-, 3-, 4-, 5-, 6-, 7-, et 8 et le radical 2-naphtyle en position 1-, 3-, 4-, 5-, 6-, et 7.

Le groupement (C₅-C₁₄)-aryle peut représenter également un système aromatique monocyclique ou polycyclique dans lequel 1,2,3 ou 4 atomes de carbone du cycle sont remplacés par des hétéroatomes, en particulier identiques ou différents du groupe constitué de l'azote, l'oxygène et le soufre. Parmi les groupements (C₅-C₁₄)-aryle hétérocycliques (= (C₅-C₁₄)-hétéroaryle) on peut citer les groupements 2-pyridyle, 3-pyridyle, 4-pyridyle, pyrrolyle, furyle, thiényle, imidazolyle, pyrazolyle, oxazolyle, isoxazolyle, thiazolyle, isothiazolyle, tétrazolyle, pyridyle, pyrazinyle, pyrimidinyle, indolyle, isoindolyle, indazolyle, phtalazinyle, quinolyle, isoquinolyle, quinoxalinyle, quinazolinyle, cinnolinyle, β-carbolinyle, ou encore des dérivés benzo-condensés, cyclopenta-, cyclohexa-, ou cyclohepta- condensés de ces radicaux.

Le système hétérocyclique peut être substitué par les mêmes substituants cités plus haut pour le système carbocyclique.

Bien entendu, le description ci-dessus concernant les groupes aryles s'applique également lorsque aryle est un radical inclus dans un groupement tel que aryl-alkyle. Comme exemples préférés de groupements aryl-alkyle, on peut citer benzyle, 1-phenylethyl ou 2-phényléhyle.

Par hétérocycle, on entend de préférence un radical à 5 ou chaînons, non aromatique, renfermant éventuellement une ou deux doubles liaisons et un ou plusieurs atomes d'azote ou d'oxygène substitué ou non substitué par les mêmes substituants cités plus haut pour le système carbocyclique ainsi que le radical oxo. L'invention comprend ainsi les hétérocycles suivants : 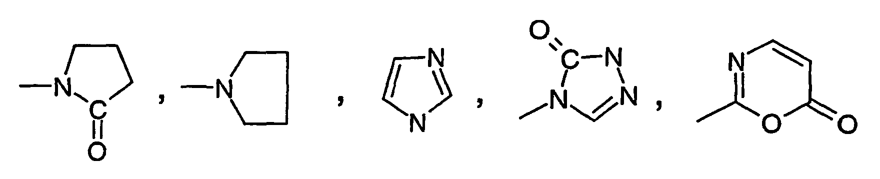 Ces hétérocycles pouvant être substitués. Il peut alors s'agir des radicaux suivants : 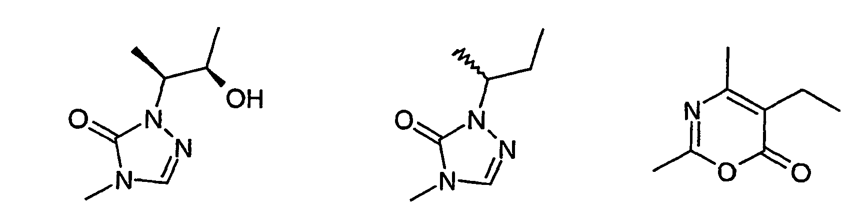 Les atomes de carbone optiquement actifs contenus dans les composés de formule (I) peuvent indépendamment les uns des autres présenter la configuration R ou la configuration S.

Les composés de formule (I) peuvent être sous la forme d'énantiomères purs ou de diastéréoisomères purs ou sous la forme d'un mélange d'énantiomères, par exemple sous forme de racémates ou de mélanges de diastéréoisomères.

La présente invention a donc pour objet les énantiomères purs, les mélanges de ces énantiomères, les diastéréoisomères purs et les mélanges de ces diastéréoisomères.

L'invention comprend les mélanges de deux ou plus de deux stéréoisomères de formule (I) et tous les rapports de ces stéréoisomères au sein des dits mélanges.

Les composés de formule (I) peuvent le cas échéant être présent sous forme d'isomères E ou d'isomères Z. L'invention a donc pour objet les isomères E purs, les isomères Z purs et les mélanges E/Z selon un rapport quelconque. Lorsque les composés de formule (I) renferment un cyclopropane, ces composés de formule (I) peuvent être présent sous forme d'isomères cis ou trans. L'invention a donc pour objet les isomères cis purs, les isomères trans purs et les mélanges cis/trans selon un rapport quelconque.

L'invention comprend également toutes les formes tautomères des composés de formule (I).Les diastéréoisomères, incluant les isomères E/Z (ou cis/trans) peuvent être séparés en isomères individuels, par exemple par chromatographie. Les racémates peuvent être séparés en deux énantiomères par des méthodes courantes telles que la chromatographie en phase chirale ou par des méthodes de résolution.

Les sels physiologiquement acceptables des composés de formule (I) sont en particulier des sels pharmaceutiquement utilisables ou non toxiques ou physiologiquement utilisables.

Lorsque les composés de formule (I) renferment un groupe acide tel que l'acide carboxylique, il s'agit par exemple des sels de métaux alcalins ou alcalino terreux tels que les sels de sodium, de potassium, de magnésium, de calcium, et également les sels formés avec des ions ammonium quaternaires physiologiquement acceptables et les sels d'addition avec les acides tel que l'ammoniac et des amines organiques physiologiquement acceptables telles que par exemple la triéthylamine, l'éthanolamine ou le tris-(2-hydroxyéthyle)amine.

Lorsque les composés de formule (I) contiennent un groupe basique, ils peuvent former un sel d'addition avec les acides par exemple avec les acides inorganiques tels que l'acide chlorhydrique, sulfurique, phosphorique ou avec les acides organiques carboxyliques tels que l'acide acétique, trifluoroacétique, citrique, benzoique, maléique, fumarique, tartarique, methanesulfonique ou para toluène sulfonique.

Les composés de formule (I) qui comportent un groupe basique et un groupe acide, tel que par exemple guanidino et carboxylique, peuvent être présents sous forme de Zwiterions (bétaïnes), qui sont également inclus dans la présente invention.

Lorsque les composés de formule (I) renferment un groupement ammonium chargé, le contre anion (Q⁻) est de préférence un anion monovalent ou un équivalent d'anion polyvalent d'un acide organique ou inorganique non toxique, physiologiquement acceptable et en particulier pharmaceutiquement acceptable, par exemple l'anion ou un équivalent d'anion d'un des acides cités plus haut utile pour la formation des sels d'addition. Q⁻ peut être par exemple un des anions (ou équivalent d'anion) d'un groupe choisi parmi chlore, sulfate, phosphate, acétate, trifluoroacétate, citrate, benzoate, maléate, fumarate, tartrate, méthanesulfonate et para-toluènesulfonate.

Les sels des composés de formule (I) peuvent être obtenus par les méthodes ordinaires connues de l'homme du métier, par exemple en combinant un composé de formule (I) avec un acide organique ou inorganique ou une base dans un solvant ou un dispersant ou à partir d'un autre sel par échange de cation ou d'anion.

L'invention inclut également tous les sels des composés de formule (I) qui, à cause de leur faible acceptabilité physiologique, ne sont pas directement utilisable comme médicament, mais sont utilisables comme produits intermédiaires pour mettre en oeuvre des modifications chimiques ultérieures au niveau des composés de formule (I) ou comme produits de départ pour la préparation de sels physiologiquement acceptables.

La présente invention inclut également tous les solvates des composés de formule (I) par exemples les hydrates, les solvates formés avec les alcools, et tous les dérivés des composés de formule (I), par exemple les esters, prodrogues et autres dérivés physiologiquement acceptables, ainsi que les métabolites des composés de formule (I).

L'invention a également pour objet les prodrogues des composés de formule (I) qui peuvent être transformées en composés de formule (I) in vivo dans les conditions physiologiques. Les prodrogues des composés de formule (I), à savoir les dérivés chimiquement modifiés des composés de formule (I) afin d'obtenir des propriétés améliorées de manière désirée, sont connus de l'homme du métier.

Pour avoir plus d'information sur le type de prodrug envisagé dans la présente invention, on peut citer les ouvrages suivants : Fleicher et al., Advanced Drug Delivery Review 19 (1996) 115-130; Design of prodrugs, H. Bundgaard, Ed., Elsevier, 1985; H. Bungaard, Drugs of the Future 16 (1991) 443; Saulnier et al. Bioorg. Med. Chem. Lett. 4 (1994) 1985; Safadi et al. Pharmaceutical Res. 10 (1993) 1350. Parmi les prodrugs appropriées des composés de formule (I) on peut citer de préférence :
- les prodrogues sous forme d'esters des groupes carboxyliques, sulfonique ou phosphonique, lorsque, par exemple, Ar³ est substitué respectivement par un groupement -CO₂H, -SO₃H ou -PO₃H.
- les prodrogues sous forme d'acyle et de carbamate pour les groupes contenant un azote acylable tel que les groupes amino ou guanidine.
- Les prodrogues sous forme de dérivés quaternaires de N tel que benzyle substitué.
Dans les prodrogues acylés ou sous forme de carbamate, une ou plusieurs fois, par exemple deux fois, un atome d'hydrogène situé sur l'atome d'azote est remplacé par un groupe acyle ou carbamate. Parmi les groupes acyles ou carbamates préférés, on peut citer les groupes R₁₂CO-, R₁₃OCO-, dans lesquels R₁₂ est un hydrogène ou un radical (C₁-C₁₈)-alkyle, (C₃-C₁₄)-cycloalkyle, (C₃-C₁₄)-cycloalkyle-(C₁-C₈)-alkyle, (C₅-C₁₄)-aryle, dans lequel 1 à 5 atomes de carbone peuvent être remplacés par des hétéroatomes tels que N,O,S ou (C₅-C₁₄)-aryle-(C₁-C₈)alkyle, dans lequel 1 à 5 atomes de carbone dans la partie aryle peuvent être remplacés par des hétéroatomes tels que N,O,S et R₁₃ à les mêmes valeurs que R₁₂ à l'exception d'hydrogène.

Bien entendu, Ar¹, Ar³, R¹, R², R³, R⁴, R⁵, R⁶, R⁷, R⁸, R⁹, R¹⁰, R¹¹, R¹² et R¹³ peuvent adopter les définitions ci-dessus de manière indépendantes les unes par rapport aux autres.

Parmi les définitions concernant la formule (I), on peut citer les valeurs préférées suivantes :
Ar¹ et Ar³ sont de préférence des phényles,
Ar¹ représente un groupement phényle et Ar³ représente un hétérocycle,
A est de préférence un hydrogène ou représente un groupement CH₂ lié à R¹ par une liaison carbone carbone cyclique pour former avec le phényle accolé, une tétrahydroisoquinoleine
B est de préférence un groupement -CH₂-CH=CH- ou -CH₂-(cyclopropyle)-, les dits groupements étant non substitués ou substitués par un ou plusieurs halogènes ou (C₁-C₄)-alkyle,
R¹ est de préférence un atome d'hydrogène ou un groupement méthyle ou éthyle non substitué ou substitué par un groupement fluor, OH, NH₂, (C₁-C₈)-alkyloxy, (C₁-C₈)-alkylamino, pyrrolidino, 2-oxopyrrolidino, ou di-(C₁-C₈)-alkylamino, ou bien représente un groupement CH₂ lié à A par une liaison carbone carbone cyclique pour former avec le phényle accolé une tétrahydroisoquinoleine,
R² et R³ sont de préférence des atomes d'halogène
R⁴ est de préférence un atome d'hydrogène
R⁶ est de préférence un atome d'hydrogène
R⁵ et R⁷ représentent de préférence hydrogène
R⁸, R⁹ et R¹⁰ représentent de préférence hydrogène, halogène, -CF₃, CN, -OCF₃, -OH, -SO₃H, -P(O) (OH)₂, carboxy, -OSO₃H, -OPO₃H, -NH₂, (C₁-C₆)-alkyle, un radical hétérocyclique saturé ou insaturé non aromatique, amino-(C₁-C₆)-alkyle, hydroxy- (C₁-C₆)-alkyle, (C₁-C₆)-alkyloxy, (C₁-C₆) -alkylamino-(C₁-C₆)-alkyloxy, (C₁-C₆)-alkyloxycarbonyle, (C₁-C₆)-alkylcarbonyle, (C₁-C₆)-alkylaminocarbonyle, (C₁-C₆)-alkylamino, di-(C₁-C₆)-alkylamino ou di-(C₁-C₆)-alkylamino-(C₁-C₆)-alkyloxy, les dits radicaux alkyles étant non substitués ou substitués par halogène, OH, SO₃H, P(O) (OH)₂, Carboxy, - OSO₃H, -OPO₃H₂, -NH₂, phényle, (C₁-C₆)-alkyloxy, (C₁-C₆)-alkylamino ou di-(C₁-C₆)-alkylamino.

L'invention a plus particulièrement pour objet les composés de formule (I) tels que définis plus haut dans laquelle B est un groupement -CH₂-CH=CH- ou -CH₂-(cyclopropyle)-, les dits groupements étant non substitués ou substitués par un ou plusieurs halogènes ou (C₁-C₄)-alkyle, Ar¹ représente un phényle disubstitué par R² et R³, ainsi que leurs sels d'addition physiologiquement acceptables.

L'invention a plus particulièrement pour objet les composés de formule (I) tels que définis plus haut répondant à la formule : 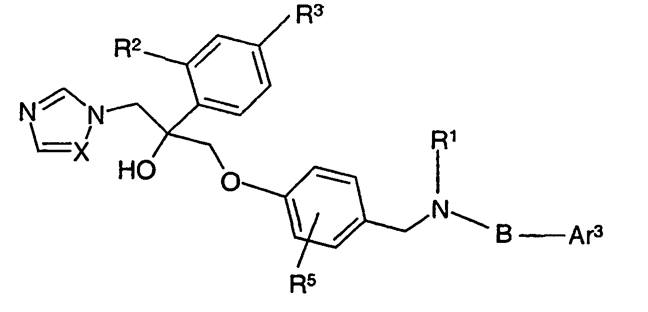 dans laquelle, B, X, Ar³, R⁵ et R¹ sont tels que définis plus haut, R² et R³ représentent un atome de chlore ou de fluor ainsi que leurs sels d'addition physiologiquement acceptables.

L'invention a plus particulièrement pour objet les composés de formule (I) tels que définis plus haut répondant à la formule (Ib) : 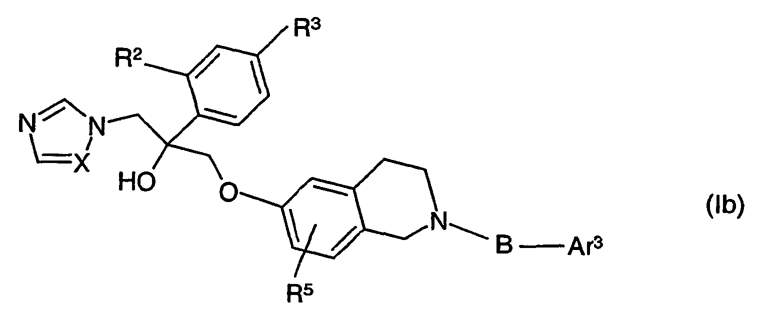 dans laquelle, B, X, Ar³, R⁵ et R¹ sont tels que définis plus haut, R² et R³ représentent un atome de chlore ou de fluor, ainsi que leurs sels d'addition physiologiquement acceptables.

L'invention a plus particulièrement pour objet les composés de formules (I), (IA) ou (IB) telles que définies plus haut dans lesquelles R₂ et R₃ sont des atomes de fluor ou de chlore, X représente CH ou NH, et Ar³ représente un groupement phényle non substitué ou substitué par R⁸ tel que défini précédemment, ainsi que leurs sels d'addition physiologiquement acceptables.

L'invention a tout particulièrement pour objet les composés de formules (I) ou (IA) telle que définies précédemment dans lesquelles, R¹ est un atome d'hydrogène ou un groupement méthyle ou éthyle non substitué ou substitué par un groupement F, OH, NH₂, (C₁-C₆)-alkyloxy, (C₁-C₆)-alkylamino, pyrrolidino, 2-oxopyrrolidino, ou di-(C₁-C₆)-alkylamino, ainsi que leurs sels d'addition physiologiquement acceptables.

L'invention a tout particulièrement pour objet les composés de formules (I), (IA) ou (IB) telles que définies précédemment dans laquelle Ar³ est un phényle non susbtitué ou substitué par R⁸ représentant un radical -Cl, -F, CN, -CF₃, -OCF₃, -OH, -NH₂, (C₁-C₆)-alkyloxy, (C₁-C₆)-alkylamino, ou di-(C₁-C₆)-alkylamino ou un hétérocycle choisi parmi :

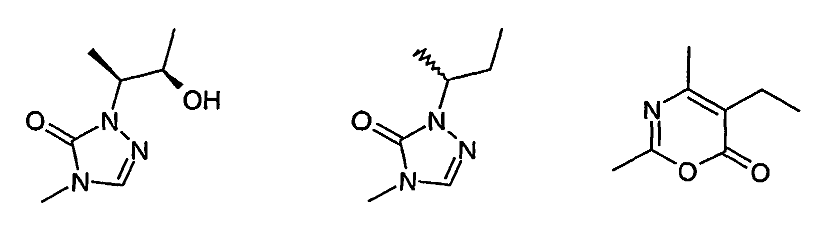

L'invention a tout particulièrement pour objet les composés suivants :
- alpha-[[[2-[3-(4-chlorophényl)-2(E)-propényl]-1,2,3,4-tétrahydro-6-isoquinoleinyl]oxy]méthyl]-alpha-(2,4-dichlorophényl)-1H-imidazol-1-éthanol
- alpha-(2,4-dichlorophényl)-alpha-[[4-[[[méthyl(3-phényl-2(E)-propényl)]amino]méthyl]phénoxy]méthyl]-1H-imidazol-1-éthanol
- alpha-(2,4-difluorophényl)-alpha-[[4-[[méthyl(3-phényl-2(E)-propényl)amino]méthyl]phénoxy]méthyl]-1H-1,2,4-triazol-1-éthanol
- alpha-[[[2-[3-(4-chlorophényl)-2(E)-propényl]-1,2,3,4-tétrahydro-6-isoquinoleinyl]oxy]méthyl]-alpha-(2,4-difluorophényl)-1H-1,2,4-triazol-1-éthanol
- alpha-(2,4-difluorophényl)-alpha-[[4-[[(2-aminoéthyl)[3-(4-chlorophényl)-2(E)-propényl]amino]méthyl]phénoxy]méthyl]-1H-imidazol-1-éthanol.

L'invention a également pour objet un procédé de préparation des composés de formule (I) caractérisé en ce que l'on soumet un composé de formule (II) 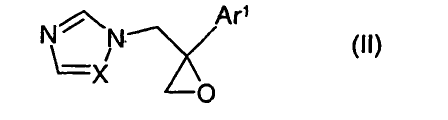 dans laquelle X et Ar1 sont tels que défini précédemment à l'action d'un composé de formules (IIIa) ou (IIIb) 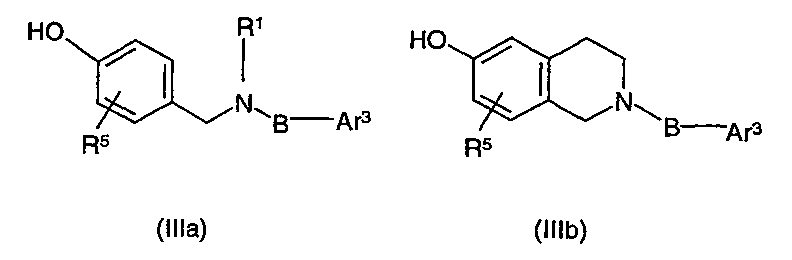 dans laquelle R¹, B, R⁵ et Ar³ conservent leur précédente signification, en milieu basique, pour obtenir le composé de formule (IA) ou (IB) correspondant.

Cette réaction s'effectue de préférence en présence de K₂CO₃ dans le DMF, et éventuellement avec un éther couronne du type 18-C-6.

A titre de variante de procédé, on fait réagir le composé de formule (II) avec un aryle de formule (III') HO-C₆H₄-CHO en présence d'une base, le phényle étant non substitué ou substitué par R⁵ afin d'obtenir un composé de formule (IIa) 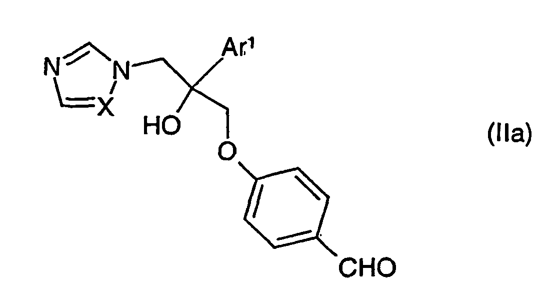 que l'on fait réagir avec une amine de formule R¹-NH₂, R¹ étant tel que défini précédemment, dont les fonctions réactives sont éventuellement protégée, suivi d'une réaction de réduction en présence d'un agent réducteur tel que NaBH₃CN ou BH₃.pyridine, afin d'obtenir un composé de formule (IIb) 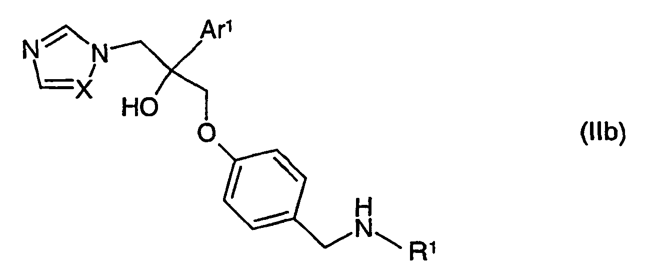 que l'on fait réagir
- soit avec un dérivé de formule

   OHC-CH=CH-C₆H₄-R⁸ ou OHC-(Cyclopropyle)-C₆H₄-R⁸

   suivi d'une réaction de réduction en présence d'un agent de réduction tel que NaBH₃CN
- soit avec un composé de formule :

   - AcO-CH₂-CH=CH-Ph-R⁸

   en présence d'un dérivé du palladium
   afin d'obtenir les composés de formules (IAA)ou(IAB) suivantes :

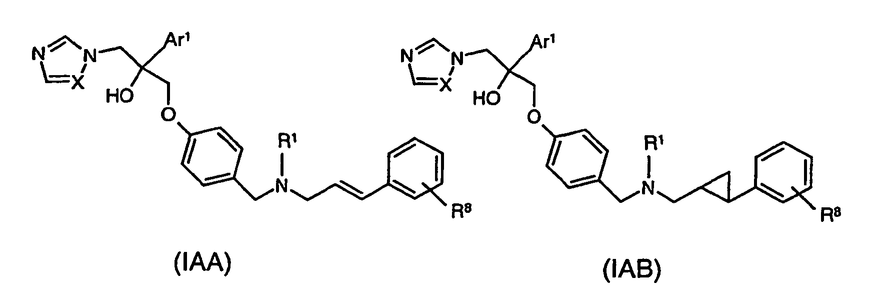

La première réaction d'amination réductrice mettant en jeu l'aldéhyde (IIa) s'effectue de préférence en présence d'un réactif tel que le NaBH₃CN dans le méthanol ou BH₃.pyridine. La deuxième réaction d'amination réductrice mettant en jeu l'amine (IIc) avec un dérivé de trans cinnamaldéhyde, s'effectue également de préférence en présence de NaBH₃CN dans le méthanol. La réaction mettant en jeu l'amine (IIc) avec un acétate allylique s'effectue en présence d'un dérivé du palladium, par exemple en milieu acétonitrile/eau (tppts/Pd(OAc)₂).

Les composés de départs de formule (II) ou (III) peuvent être préparés selon des procédés décrits dans la littérature ou encore sont accessibles par analogie. La préparation des composés de formule (II) s'effectue selon le schéma suivant : 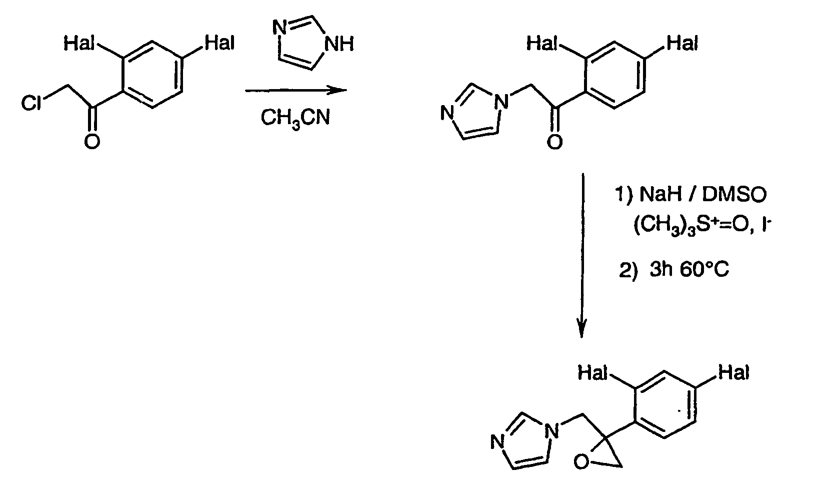 Certains composés de formule (III) (R¹ = Me) sont aisément accessibles. Ils peuvent être préparés comme indiqué dans le schèma ci-dessous ou dans la partie expérimentale. 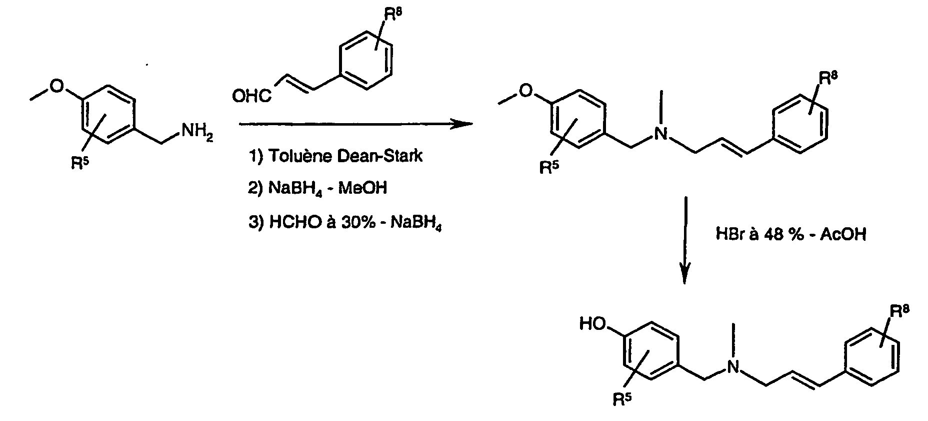 les composés de formule III tels que le (E)-2-[3-phényl)-2-propényl]-1,2,3,4-tetrahydro-6-isoquinolinol ou ses dérivés (phényle substitué par R⁸) sont préparés selon la méthode décrite dans WO 00/20413.

Etant entendu que la présente invention n'est pas restreinte à ces synthèses ou à ces produits de départ. Il n'y a pas de difficulté majeure pour l'homme du métier de prévoir des modifications des synthèses décrites dans notre demande pour la préparation d'autres composés de formule (I) selon l'invention.

Les composés de formule (I) sont des composés ayant une activité pharmacologique et peuvent ainsi être utilisés à titre de médicaments notamment comme antifongiques.

La présente invention a donc pour objet les composés de formule (I) et/ou leurs sels physiologiquement acceptables et/ou leurs pro-drogues à titre de médicament.

Les composés de formule (I) ainsi que leurs sels physiologiquement acceptables et leurs prodrugs peuvent être administrés aux animaux, de préférence aux mammifères et en particulier aux êtres humains comme médicaments à titre thérapeutique ou prophylactique.

Les composés de formule (I) présentent d'intéressantes propriétés antifongiques. Ils sont notamment actifs sur Candida albicans et autres Candida comme Candida glabrata, krusei, tropicalis, pseudotropicalis et parapsilosis, sur Aspergillus, Aspergillus flavus, Aspergillus niger, Cryptococcus néoformans, Microsporum canis, Trichophyton rubrun, Trichophyton mentagrophyte.

Les composés de formule (I) peuvent être utilisés en tant que médicament chez l'homme ou l'animal, pour lutter notamment contre les candidoses digestives, urinaires, vaginales ou cutanées, les cryptococcoses, par exemple les cryptococcoses neuroméningées, pulmonaires ou cutanées, les aspergilloses bronchopulmonaires et pulmonaires et les aspergilloses invasives de l'immunodéprimé.

Les composés selon l'invention peuvent être utilisés également dans la prévention des affections mycosiques chez les déprimés immunitaires congénitaux ou acquis.

Les composés de l'invention ne sont pas limités à une utilisation pharmaceutiques. Ils peuvent être également utilisés comme fongicides dans d'autres domaines que pharmaceutiques.

L'invention a donc pour objet à titre de médicaments antifongiques, les composés de formule (I) .

Les composés selon l'invention peuvent être administrés tels quels ou en mélange avec un ou plusieurs autres composés de formule (I) ou encore sous la forme d'une préparation pharmaceutique (composition pharmaceutique) qui permet une administration entérale ou parentérale et qui renferme à titre de composé actif une dose efficace d'au moins un composé de formule (I) et/ou ses sels physiologiquement acceptables et/ou ses prodrugs ainsi que des supports et/ou additifs courants et pharmaceutiquement inertes.

Les compositions pharmaceutiques selon l'invention permettent une administration entérale ou parentérale qui renferment à titre de composé actif une dose efficace d'au moins un composé de formule (I) et/ou ses sels physiologiquement acceptables et/ou ses prodrugs ainsi qu'un ou plusieurs supports pharmaceutiquement inertes, et/ou un ou plusieurs additifs usuels.

L'invention a donc pour objet les compositions pharmaceutiques renfermant un composé de formule (I) tel que défini précédemment ainsi qu'un véhicule.

Les médicaments peuvent être administrés oralement, par exemple sous forme de pilule, de comprimés, de comprimés enrobés, de pelliculés, de granules, de gélules et capsules molles, de solutions, de sirops, d'émulsion, de suspension ou de mélange d'aérosol.

L'administration peut cependant être effectuée par voie rectale, par exemple sous forme de suppositoire, par voie parentérale, par exemple sous forme de solutions injectables ou d'infusions, de microcapsules ou d'implants, par voie percutanée, par exemple sous la forme de pommade, de solutions, de pigments ou de colorants, par voie transdermique sous forme de patches ou par d'autres voies telles que sous la forme d'aérosol ou de spray nasal.

Les compositions pharmaceutiques selon l'invention sont préparées selon des méthodes connues en soi, des supports organiques ou inorganiques, pharmaceutiquement inertes étant additionnés aux composés de formule (I) et/ou leurs sels physiologiquement acceptables et/ou leurs prodrugs. Pour la production de pilule, de comprimés, de comprimés enrobés et de capsule en gélatine dure, il est possible d'utiliser par exemple, du lactose, de l'amidon de maïs ou ses dérivés, du talc, de l'acide stéarique ou ses sels, etc. Les supports convenables pour des capsules en gélatine molle ou pour les suppositoires sont par exemple les graisses, les cires les polyols semi-solides ou liquides, les huiles naturelles ou modifiées etc. Les véhicules appropriés pour la préparation de solutions, par exemple les solutions injectables, les émulsions ou les sirops sont par exemple l'eau, les alcools, le glycérol, les polyols, le sucrose, les sucres invertis, le glucose, les huiles végétales, etc. Les supports convenables pour les microcapsules ou les implants sont par exemple les copolymères d'acide glyoxilique et d'acide lactique. Les préparations pharmaceutiques contiennent normalement de 0,5% à 90% en poids de composés de formule (I) et/ou leurs sels physiologiquement acceptables.

En plus des principes actifs et des supports, les préparations pharmaceutiques peuvent contenir des additifs tels que par exemple des diluants, des désintégrants, des liants, des lubrifiants, des agents mouillant, des stabilisants, des émulsifiants, des préservateurs, des agents sucrant, des colorants des agents de flaveurs ou des aromatisants, des épaississants, des agents tampons, et aussi des solvants ou des solubilisants ou des agents pour obtenir un effet retard et également des sels pour modifier la pression osmotique, des agents d'enrobage ou des antioxydants. Elles peuvent également contenir deux ou plusieurs composés de formule (I) et/ou leurs sels physiologiquement acceptables et/ou leurs prodrugs. En outre, en plus d'au moins un ou plusieurs composés de formule (I) et/ou leurs sels physiologiquement acceptables et/ou leurs prodrugs, ils peuvent contenir au moins un ou plusieurs autres principes actifs utilisables à titre thérapeutique ou prophylactique.

Les préparations pharmaceutiques (compositions pharmaceutiques) renferment normalement de 0,2 à 500 mg, et de préférence de 1 à 200 mg de composé de formule (I) et/ou leurs sels physiologiquement acceptables et/ou leurs prodrugs.

La présente invention a donc plus particulièrement pour objet un composé de formule (I) et/ou ses sels physiologiquement acceptables et/ou ses prodrugs tels que définis plus haut à titre de médicament ayant une activité antifongique.

La présente invention a également pour objet l'utilisation des composés de formule (I) et/ou leurs sels physiologiquement acceptables et/ou leurs prodrugs tels que définis plus haut pour la préparation de médicaments antifongiques.

Lorsqu'on utilise les composés de formule (I), les doses peuvent varier à l'intérieur de limites larges et doivent être fixées en fonction de la personne à traiter. Ceci dépend par exemple du composé employé ou de la nature et de la sévérité de la maladie à traiter et si on se trouve dans des conditions graves ou chronique ou si on met en oeuvre un traitement prophylactique.

Dans le cas d'une administration par voie orale, la dose quotidienne varie en général de 0,01 à 100mg/kg et de préférence de 0,1 à 50 mg/kg, en particulier de 0,1 à 5 mg/kg.

Dans le cas d'une administration par voie intraveineuse, la dose quotidienne varie approximativement de 0,01 à 100 mg/kg et de préférence de 0,05 à 10 mg/kg.
La dose quotidienne peut être divisée, en particulier dans le cas de l'administration de grande quantité de principe actif, en plusieurs, par exemple 2,3 ou 4 parts. Le cas échéant, en fonction du comportement individuel, il peut être nécessaire d'administrer les différentes doses de manière croissante ou décroissante.

Les composés de formule (I) et leurs sels peuvent également être utilisés comme intermédiaires pour la préparation d'autres composés, en particulier d'autres agents actifs, qui sont accessibles à partir des composés de formule (I), par exemple par modification ou introduction de radicaux ou de groupes fonctionnels.

L'invention a également pour objet à titre de composés intermédiaires les composés de formules (IIa) et (IIb) tels que définis plus haut.

### Exemples

Les produits ont été identifiés par spectre de masse (MS), infrarouge (IR) et/ou spectre RMN. Les composés ont été purifiés par chromatographie en phase normale (notamment en présence du mélange CH₂Cl₂/MeOH) ou en phase inverse (en présence d'acide acétique ou trifluoroacétique). Les composés de formule (I) purifiés en utilisant un éluant qui contient par exemple de l'acide trifluoroacétique, et qui ensuite sont séchés ou dans lesquels, lors de la dernière étape de synthèse, par exemple de l'acide trifluoroacétique a été utilisé pour éliminer un groupe protecteur tert-butyl, contiennent parfois, en fonction de la manière dont le produit a été séché, l'acide provenant de l'éluant ou de la dernière étape de synthèse et donc se trouvent partiellement ou complètement sous la forme du sel de l'acide utilisé, par exemple sous la forme d'un sel d'acide acétique ou trifluoroacétique. Ils peuvent également être plus ou moins hydratés.

### Abbrévations/noms chimiques éventuellement employés:

AcOEt : acétate d'éthyle ; DMF : diméthylformamide ; HOBt : 1-hydroxybenzotriazole hydrate, MeOH : méthanol ; TEA : triéthylamine ; TFA : acide trifluoroacétique ; THF : tétrahydrofurane ; MCPBA : acide méta-chloroperoxybenzoique ; DBU : 1,8-diazabicyclo[5.4.0]undec-7-ene; APTS : acide paratoluènesulfonique ; DPPA : diphenylphosphorylazide; DMSO: diméthylsulfoxyde ; Pd/C Palladium sur charbon ; Boc : terbutoxycarbonyl ; CBz : benzyloxycarbonyl ; DCC 1,3-dicyclohexylcarbodiimide ; IR : Infrarouge ; RMN : Résonnance Magnétique Nucléaire ; SM: Spectre de Masse ; ESP : Electrospray mode positif ; ep. : Épaulement ; F : fort ; s : singulet ; d : doublet ; t: triplet ; quad : quadruplet ; quint : quintuplet ; 1: large ; m : multiplet ou massif; J : constante de couplage ; Rf : facteur de rétention (chromatographie); Cq : Carbone quaternaire.

Les spectres RMN ci-dessous ont été interprétés et les hydrogènes aromatiques sont identifiées ainsi

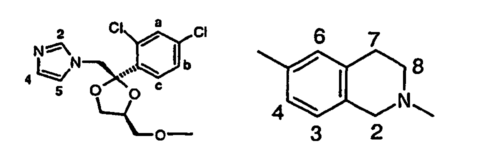

### Préparation 1

### a) Substitution (introduction de l'imidazole)

### 1-(2,4-dichlorophényl)-2-(1H-imidazol-1-yl)-éthanone

A 5,10 g d'imidazole (75 mmoles) dans 25 ml d'acétonitrile, on ajoute 5,58 g de dérivé trichloré (2-chloro-1-(2,4-dichlorophényl)-éthanone) et agite à température ambiante 24 heures. On ajoute ensuite 50 ml de dichlorométhane et 50 ml d'eau, extrait, décante, lave puis ré-extrait avec du dichlorométhane. Les phases organiques sont séchées et évaporées sous pression réduite jusqu'à obtention d'un extrait sec que l'on purifie par chromatographie sur silice en éluant avec le mélange CH₂Cl₂ /MeOH 96 /4 . On obtient 4,76 g de produit attendu.
RMN CDCl₃ 300 MHz
5,35 (s, 2H, N-CH₂-CO) ; 6,95 (s) et 7,13 (s) 2H H4 et H5; 7,38 (dd, 1H, Hb) ; 7,51 (d, 1H, Ha) ; 7,56 (sl, 1H, H2) ; 7,58 (d, 1H, Hc)

### b) Formation de l'époxyde

### 1-[[2(2,4-dichlorophényl)oxiranyl]méthyl]-1H-imidazole (P1)

A 1,32 g de Iodure de trimétylsulfoxonium (6 mmoles) dans 15 ml de DMF, on ajoute 0,317 g-déhydrure de sodium (6,6 mmoles) puis 1,53 g du dérivé d'imidazole (6 mmoles) et agite 5 heures à 70°C - 75°C. On verse ensuite le milieu réactionnel dans de l'eau, extrait avec de l'éther, sèche puis évapore sous pression réduite pour obtenir le produit brut sous forme d'une huile que l'on purifie par chromatographie en éluant avec un mélange CH₂Cl₂/MeOH 95/5. On obtient 0,853 g de produit attendu.
RMN CDCl₃ 300 MHz
2,86 et 2,95 (AB, 2H, O-CH₂-Cq) ; 4,12 et 4,66 (AB, 2H, N-CH₂-Cq) ; 6,89 (sl) et 7,02 (sl) 2H H4 et H5; 7,09 (d, 1H, Hc) ; 7,16 (dd, 1H, Hb) ; 7,39 (sl, 1H, H2) ; 7,41(d, 1H, Ha).

### Préparation 2

### a) Substitution (introduction de l'imidazole)

### 1-(2,4-difluorophényl)-2-(1H-1,2,4-imidazol-1-yl)-éthanone

A 0,612 g d'imidazole (9 mmoles) dans 3 ml d'acétonitrile, on ajoute 0,572 g de dérivé (2-chloro-1-(2,4-difluorophényl)-éthanone) et agite à température ambiante 24 heures. On extrait ensuite avec du dichlorométhane, lave, sèche et évapore sous pression réduite jusqu'à obtention du produit brut sous forme d'huile que l'on recristallise dans de l'éther . On obtient 0,521 g de produit attendu. F = 121°C.
RMN CDCl₃ 300 MHz
5,32 (m, 2H, CO-CH₂-N) ; 6,94 (sl) et 7,15 (sl) : 2H H4 et H5; 6,98 (ddd, 1H, Ha) ; 7,08 (ddd, 1H, Hb) ; 7,56 (sl, 1H, H2) ; 8,05 (dt, 1H, Hc)

### b) Formation de l'époxyde

### 1-[[2-(2,4-difluorophényl)oxiranyl]méthyl]-1H-1,2,4-imidazole (P2)

On ajoute 0,211 g (44 mmoles) de NaH à 50% à 0,88 g (4mmoles) de Iodure de trimétylsulfoxnium dans le DMSO (10 ml), agite 30 mn puis ajoute par portion 0,889 g (4 mmoles) de dérivé d'imidazole préparé au stade précédent et chauffe 3 heures à 60°C. On ajoute 20 ml d'eau, extrait avec de l'éther, sèche sur Na₂SO₄, filtre et évapore sous pression réduite pour obtenir le produit brut (1,16g) sous forme d'une huile que l'on purifie par chromatographie en éluant avec un mélange dichlorométhane/méthanol 97/3. On obtient 0,52 g de produit attendu (Rf = 0,3 . dichlorométhane/méthanol 97/3). RMN CDCl₃ 300 MHz
2,85 et 2,94 (AB, 2H, OCH₂-) ; 4,11 et 4,62 (AB, 2H, N-CH₂) ; 6,90 (s) 6,99 (s) H4 et H5 ; 7,38 (s) H2 ; 6,81 (m, 2H) 7,14 (m, 1H) : Ha, Hb, Hc.

### Préparation 3

### Formation de l'époxyde

### 1-[[2(2,4-difluorophényl)oxiranyl]méthyl]-1H-1,2,4-triazole (P3)

On agite pendant 3 heures à 70°C le mélange constitué de 2,23 g de dérivé de Triazole (1-(2,4-difluorophényl)-2-(1H-1,2,4-triazol-1-yl)-éthanone)(Commercial : D et O Pharmachem), 15 ml de soude 5N, 2,2 g de Iodure de trimétylsulfonium (10 mmoles) et 0,088 g de cétrimide (Jansen) dans 15 ml de DMF. On évapore ensuite sous pression réduite pour obtenir le produit brut sous forme d'une huile que l'on purifie par chromatographie en éluant avec un mélange acétate d'éthyle/méthanol 7/3. On obtient 0,689 g de produit attendu.
RMN CDCl₃ 300 MHz
2,90 et 2,97 (AB, 2H, O-CH₂-Cq) ; 4,53 et 4,86 (AB, 2H, Cq-CH₂-N) ; 7,19 (td, 1H, Hc) ; 6,83 (m, 2H, Ha et Hb) ; 7,91 (s) et 8,20 (s, 2H, N=CH-N) .

### Préparation 4

### 4-hydroxy-N-méthyl-N-(3-phényl-2(E)-propényl)-benzène-méthanamine (P4)

### Stade a) : 4-méthoxy-N-méthyl-N-(3-phényl-2(E)-propényl)-benzèneméthanamine

On chauffe au reflux pendant 2 heures 30 minutes une solution constituée de trans-cinnamaldehyde (Jansen, d=1,048, 13,2 g, 0,1 mol) et de 4-methoxybenzylamine (Fluka, d=1,057, 13,7 g, 0,1 mol) dans 250 ml de toluène, tout en éliminant l'eau formée au cours de la réaction à l'aide d'un « Dean-Starck », puis évapore sous pression réduite le toluène. Le résidu obtenu (base de Schiff) est ensuite solubilisé dans 150 ml de méthanol puis on réduit la base de Schiff en ajoutant à 40°C 3,8 g de NaBH₄. On ajoute enfin au milieu réactionnnel 81 ml de formaldehyde à 37% (réaction d'amino réduction), porte le mélange au reflux pendant 30 minutes et agite une nuit à température ambiante. Après évaporation du méthanol, on reprend le résidu avec du dichlorométhane, lave deux fois à l'eau et une fois avec une solution aqueuse saturée en NaCl, sèche sur MgSO₄, filtre et évapore sous pression réduite jusqu'à obtention d'un extrait sec que l'on repurifie par chromatographie sur silice en éluant avec un mélange CH₂Cl₂/AcOEt 70/30. On obtient 9,07 g de produit attendu cristallisé. Rf 0,20 CH₂Cl₂/AcOEt 70/30.
RMN ¹H (300MHz CDCl₃) 2,23 (s, 3H, CH₃-N) ; 3,18 (dl, 2H, N-CH₂-CH=CH-Ph) ; 6,31 (td, J= 16; 6,5 Hz, N-CH₂-CH=CH-Ph) ; 6,54 (d, 1H, J = 16 Hz, N-CH₂-CH=CH-Ph) ; 3,49 (s, 2H, Ph-CH₂-N) ; 3,80 (s, 3H, Ph-O-CH₃) ; 6,86 et 7, 25 AA'BB' ; 7,31 (tl, 2H, H méta) ; 7,38 (dl, 2H, H ortho) ; 7,24 (masqué 1H, H para).

### Stade b) : 4-hydroxy-N-méthyl-N-(3-phényl-2(E)-propényl)-benzèneméthanamine.

A une solution de produit préparé au stade précédent ( 1 g, 3,74 mmol) dans 20 ml d'acide acétique, on ajoute 20 ml d'acide bromhydrique à 48 % et chauffe au reflux pendant 5 heures et 30 minutes. Après évaporation sous pression réduite en entraînant l'eau avec de l'acétate d'éthyle, on obtient un extrait sec que l'on purifie par chromatographie sur silice en éluant avec le mélange CH₂Cl₂/MeOH 95/5 pour obtenir 660 mg de produit attendu. Rf 0,46 CH₂Cl₂/MeOH 95/5.

### Exemple 1 : alpha-[[[2-[3-(4-chlorophényl)-2(E)-propényl]-1,2,3,4-tétrahydro-6-isoquinoleinyl]oxy]méthyl]-alpha-(2,4-dichlorophényl)-1H-imidazol-1-éthanol

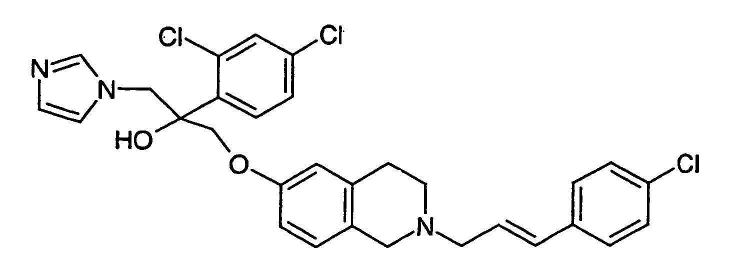

On agite pendant 32 heures à 80°C le mélange constitué de (P1) (0,17 g), de (2-[3-(4-chlorophényl)-2(E)-propényl]-1,2,3,4-tétrahydro-6-isoquinoléinol) (0,18 g) (préparé selon WO 00/20413), de 18-C-6 (17 mg) et de K₂CO₃ (0,341 g) dans 2 ml de DMF puis extrait avec du dichlorométhane, lave à l'eau, sèche sur MgSO₄, filtre et évapore sous pression réduite pour obtenir 0,612 g de produit brut que l'on purifie par chromatographie sur silice en éluant avec le mélange CH₂Cl₂/MeOH/NH₄OH 95/5/0,3 puis acétone/heptane 1/1 puis une nouvelle fois CH₂Cl₂/MeOH/NH₄OH 95/5/0,3 pour obtenir 0,1 g de produit attendu Rf = 0,28.
¹H RMN (CDCl₃)
2,66 (m, 2H CH₂ en 7) ; 2,77 (m, 2H, CH2 en 8) ; 3,25 (dl, 2H N-CH₂-CH=CH-Ph) ; 6,39 (td, 1H J = 16, 7 Hz) ; 6,60 (dl, 1H) ; 4,50 4,77 (AB) et 4,27 4,48 (AB) : 4H, N-CH₂-Cq-CH₂-O ; 7,37 (masqué, Hb) ; 6,67 (m, 2H, H6 et H4) ; 6,94 (d, H3) ; 7,65 (d, 1H, Hc) ; 7,56 (d, 1H, Ha) ; 7,38 7,50 AA'BB' 4H H aromatiques ; 6,34 (s, 1H, OH) ; 6,70 (sl) 7,37 (sl) et 6,87 (sl) 3H H2', H4' et H5' ; 3,50 (sl, 2H, CH₂ en 2).
IR (CDCl₃)
3563 cm⁻¹ (-OH) ; 1610 cm⁻¹ (C=C) ; 1590, 1556, 1505 et 1491 cm⁻¹ (hétérocycle + aromatiques).

### Exemple 2 : alpha-(2,4-dichlorophényl)-alpha-[[4-[[[méthyl(3-phényl-2(E)-propényl)]amino]méthyl]phénoxy]méthyl]-1H-imidazol-1-éthanol

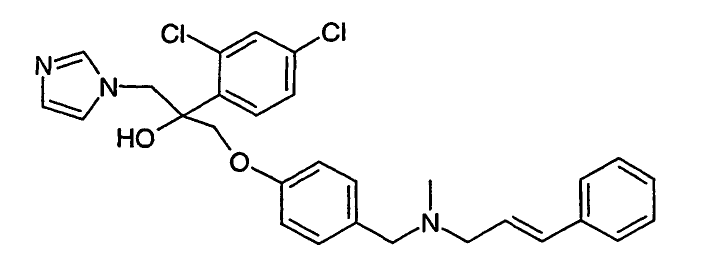

On agite pendant 5 heures à 80°C le mélange constitué de (P1) (0,152 g, 0,6 mmole), de 4-hydroxy-N-méthyl-N-(3-phényl-2(E)-propényl)-benzèneméthanamine (P4)(0,24 g, 0,9 mmole), de 18-C-6 (17 mg) et de K₂CO₃ (0, 124 g, 0,9 mmole) dans 2 ml de DMF puis extrait avec du dichlorométhane, lave à l'eau, sèche sur Na₂SO4, filtre et évapore sous pression réduite pour obtenir 0,5 g de produit brut que l'on purifie par chromatographie sur silice en éluant avec le mélange CH₂Cl₂ /MeOH / NH₄OH 95 / 5 / 0,3 pour obtenir 71 mg de produit pur attendu (pureté = 95 %). Rf = 0,2 (CH₂Cl₂ / MeOH / NH₄OH 95 /5 / 0,3)
¹H RMN (CDCl₃)
2,10 (s, N-CH₃) ; 3,11 (d, N-CH₂-CH=CH) ; 6,32 (dt, J= 16 et 7 Hz, N-CH₂-CH=CH) ; 6,54 (d, J = 16 Hz, N-CH₂-CH=CH) ; 3,43 (s, Ph-CH₂-N) ; 4,31 (d, J = 10) 4,51 (d, J= 10) : AB =C-N-CH₂-Cq; 4,52 (d, J= 14,5) 4,79 (d, J=14,5) : AB Ph-O-CH₂-Cq ; 6,86 et 7,22 (AA'BB') O-Ph ; 6,35 (s, 1H supposé mobile) ; 6,70 (s) 6,88 (s) 7,38 (s) 3H imidazole ; 7,56 (d, H2) ; 7,20 à 7,47 (m) H du phényle ; 7,37 (masqué, H6) ; 7,66 (d, H5).

### Exemple 3 : alpha-[[[2-[3-(4-chlorophényl)-2(E)-propényl]-1,2,3,4-tétrahydro-6-isoquinoleinyl]oxy]méthyl]-alpha-(2,4-difluorophényl)-1H-1,2,4-triazol-1-éthanol

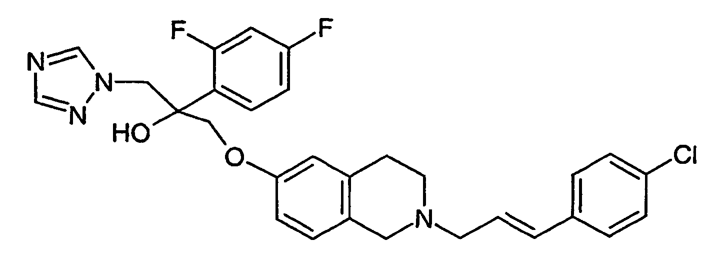

On agite pendant 2 heures à 80°C le mélange constitué de (P3) (0,2 g, 0,66 mmole), de (2-[3-(4-chlorophényl)-2(E)-propényl]-1,2,3,4-tétrahydro-6-isoquinoléinol) (préparé selon WO 00/20413) (0,144 g, 0,6 mmole), de 18-C-6 (15 mg) et de K₂CO₃ (0, 167 g, 1,2 mmole) dans 2 ml de DMF puis extrait avec du dichlorométhane, lave à l'eau, sèche sur Na₂SO4, filtre et évapore sous pression réduite pour obtenir 0,49 g de produit brut que l'on purifie par chromatographie sur silice en éluant avec le mélange CH₂Cl₂/MeOH/NH₄OH 94/6/0,3 pour obtenir 0,2 g de produit pur attendu. Rf = 0,35 (CH₂Cl₂/MeOH/NH₄OH 95/5/0,3).
¹H RMN (CDCl₃)
2,82 (m, 2H CH₂ en 7) ; 2,88 (m, 2H, CH2 en 8) ; 3,66 (sl, =C-CH₂-N ; 3,37 (dl, 2H N-CH₂-CH=CH-Ph) ; 6,34 (dt, 1H J = 16, 7 Hz, N-CH₂-CH=CH-Ph) ; 6,55 (dt, 1H J = 16 Hz, N-CH₂-CH=CH-Ph); 4,20 (AB, C-CH2-O) ; 4,85 (AB, N-CH₂-C) ; 6,62 (d, H'a) ; 6,66 (dd, H'c) ; 6,93 (d, H'b) ; 6,82 (m, Ha et Hb) ; 7,460 (dt, Hc) ; 7,83 (s) et 8,02 (s) : H3 et H5
IR (CDCl₃)
3565 cm⁻¹ (-OH) ; 1670, 1618, 1501 et 1491 cm⁻¹ ( -C=C-, hétérocycle + aromatiques).

### Exemple 4 : alpha-(2,4-difluorophényl)-alpha-[[4-[[méthyl(3-phényl-2(E)-propényl)amino]méthyl]phénoxy]méthyl]-1H-1,2,4-triazol-1-éthanol

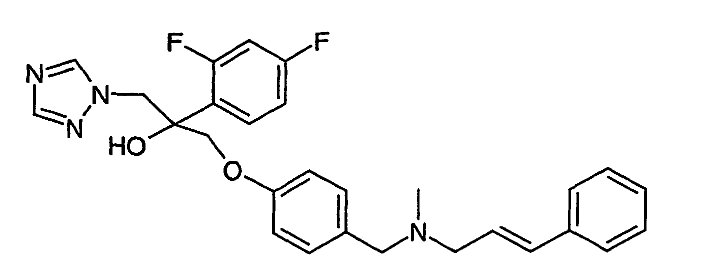

On agite pendant 2 heures à 80°C le mélange constitué de (P3) (0,145 g, 0,61 mmole), de 4-hydroxy-N-méthyl-N-(3-phényl-2(E)-propényl)-benzèneméthanamine (P4) (0,152 g, 0,6 mmole), de 18-C-6 (11 mg) et de K₂CO₃ (0,167 g, 1,2 mmole) dans 2 ml de DMF puis extrait avec du dichlorométhane, lave à l'eau, sèche sur Na₂SO4, filtre et évapore sous pression réduite pour obtenir 0,391 g de produit brut que l'on purifie par chromatographie sur silice en éluant avec le mélange CH₂Cl₂/MeOH/NH₄OH 94/6/0,3 pour obtenir 0,163 mg de produit pur attendu (pureté = 95 %). Rf = 0,25 (CH₂Cl₂/MeOH/NH₄OH 95/5/0,3).
¹H RMN (CDCl₃)
2,23 (s, N-CH₃) ; 3,18 (d, N-CH₂-CH=CH) ; 6,29 (td, J= 16 et 6,5 Hz, N-CH₂-CH=CH) ; 6,53 (dl, J = 16 Hz, N-CH₂-CH=CH) ; 3,50 (s, Ph-CH₂-N) ; 4,21 4,26 (AB, 2H) et 4,83 4,90 (AB, 2H) : =C-N-CH₂-Cq et Ph-O-CH₂-Cq ; 4,47 (1H, OH) ; 6,84 et 7,24 (AA'BB') O-Ph ; 6,85 (m, 2H) 7,61 (td 1H) 3H imidazole Ha, Hb et Hc ; 7,84 (s) et 8,03 (s) 2H H2 et H4 ; 7,20 à 7,40 (m) H du phényle .

### Exemple 5 : alpha-(2,4-difluorophényl)-alpha-[[4-[[(2-amino-éthyl) [3-(4-chlorophényl)-2(E)-propényl]-amino]-méthyl]-phénoxy]-méthyl]-1H-imidazol-1-éthanol

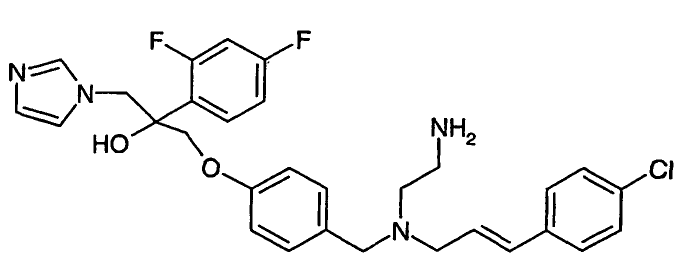

### stade a) : ouverture de l'époxyde

### 4-[2-(2,4-difluorophényl)-2-hydroxy-3-(1H-imidazol-1-yl)-propoxy]-benzaldéhyde

On agite pendant 6 heures le mélange constitué de 1-[[2-(2,4-difluorophényl)-oxiranyl]-méthyl]-1H-imidazole (P2) (0,5 g, 2,1 mmole), de 4-hydroxy-benzaldehyde (0,31 g, 2,5 mmoles) de K₂CO₃ (0,41 g, 2,9 mmoles), de 18-C-6 (25 mg) dans 5 ml de DMF, extrait avec du dichlorométhane, lave avec de la soude 2N, sèche sur Na₂SO₄, filtre et évapore sous pression réduite pour obtenir 0,957 g de produit brut que l'on purifie par chromatographie en éluant avec le mélange CH₂Cl₂/MeOH 97/3 pour obtenir 0,557 mg de produit attendu. Rf = 0,12 (CH₂Cl₂/MeOH 97/3).
¹H RMN (DMSO)
4,27 4,40 AB 2H et 4,48 (sl, 2H) : N-CH₂-Cq et 0-CH₂-Cq ; 6,37 (sl, 1H, OH) ; 6,73 (sl) et 6,91 (sl) 2H H4 et H5 ; 7,39 (sl, 1H, H2) ; 7,11 et 7,85 AA'BB' 4H -O-Ph) ; 7,53 (td, 1H, Hc) ; 7,21 (ddd, 1H, Ha) ; 7,04 (td, 1H, Hb) ; 9,86 (s, 1H, CHO).

### Stade b) Amino-réduction

### [2-[[[4-[2-(2,4-difluorophényl)-2-hydroxy-3-(1H-imidazol-1-yl)propoxy]phényl]méthyl]amino]éthyl]-carbamate de1,1-diméthyléthyle

On agite pendant 2 heures à température ambiante le mélange constitué du produit préparé au stade a (0,55 g, 1,5 mmole), de la diamine mono BOC protégée 2-aminoéthyl-carbamate de 1,1-diméthyléthyle (0,27 g, 1,68 mmole), d'acide acétique (0,128 ml) dans 5,5 ml de méthanol puis ajoute du NaBH₃CN (0,125 g, 1,98 mmole). On agite encore 2 heures à température ambiante, reprend avec du dichlorométhane, sèche sur MgSO₄, filtre puis évapore sous pression réduite pour obtenir 0,678 g de produit brut que l'on purifie par chromatographie en éluant avec le mélange CH₂Cl₂/MeOH/NH₄OH 95/5/0,3. On obtient 0,425 g de produit pur attendu.
Rf = 0,18 CH₂Cl₂/MeOH/NH₄OH 95/5/0,3.
¹H RMN (DMSO)
1,43 (s, 9H, OC(CH₃)₃ ; 2,76 (m, 2H HN-CH₂-CH₂-NH) ; 3,23 (m, 2H, HN-CH₂-CH₂-NH) ; 5,13 (sl, 1H, HN-CH₂-CH₂-NH) ; 3,76 (sl, 2H, NH-CH₂-Ph) ; 4,23 4,27 AB et 4,45 4,52 AB : N-CH₂-Cq et 0-CH₂-Cq ; 6,82 et 7,26 AA'BB' 4H -O-Ph) ; 6,80 (sl) et 6,88 (sl) 2H H4 et H5 ; 7,38 (sl, H2) ;6,85 (m, 2H, Hb et Ha) ; 7,56 (td, 1H, Hc)

### Stade c) Amino réduction

### 2-[3-(4-chlorophényl)-2(E)-propényl]-[[4-[2-(2,4-difluorophényl)-2-hydroxy-3-(1H-imidazol-1-yl)-propoxy]-phényl]-méthyl]-amino]-éthyl]-carbamate de 1,1-diméthyléthyle.

On agite pendant 2 heures à température ambiante le mélange constitué du produit préparé au stade b (0,4 g, 0,79 mmole), d'aldehyde ()(0,146 g, 0,84 mmole), d'acide acétique (68 µl) dans 5 ml de méthanol puis ajoute le NaBH₃CN (0,065 g, 1 mmole). On agite encore une nuit à température ambiante, ajoute de l'eau, ajuste le pH avec du NH₄OH concentré, extrait avec du dichlorométhane, sèche sur MgSO₄, filtre puis évapore sous pression réduite pour obtenir 0,507 g de produit brut que l'on purifie par chromatographie en éluant avec le mélange CH₂Cl₂/MeOH/NH₄OH 95/5/0,3. On obtient 0,36 g de produit pur attendu. Rf = 0,2 CH₂Cl₂/MeOH/NH₄OH 95/5/0,3.

### Stade d) Déprotection

### alpha-(2,4-difluorophényl)-alpha-[[4-[[(2-aminoéthyl)[3-(4-chlorophényl)-2(E)-propényl]amino]méthyl]phénoxy]méthyl]-1H-imidazol-1-éthanol

A 0,33 g de produit obtenu au stade c dans 5 ml de dichlorométhane, on ajoute l'acide trifluoroacétique (3,3 ml) tout en refroidissant par un bain de glace, agite 30 mn à 0-5 °C, laisse ensuite revenir à température ambiante et agite encore pendant 3 heures. Après évaporation sous pression réduite, on ajoute à l'extrait sec un mélange de dichlorométhane et d'eau, ajuste le pH à 8 avec le NH₄OH concentré, extrait avec du dichlorométhane, sèche les phases organiques avec du MgSO₄, filtre et évapore sous pression réduite pour obtenir 0,284 g de produit brut que l'on purifie par chromatographie en éluant avec le mélange CH₂Cl₂/MeOH/NH₄OH 90/10/0,3. On obtient 0,121 g de produit pur attendu. Rf = 0,25 CH₂Cl₂/MeOH/NH₄OH 90/10/0,3.
¹H RMN (CDCl₃)
2,59 (N-CH₂-CH₂-NH₂) ; 2,76 (N-CH₂-CH₂-NH₂) ; 3,25 (N-CH₂-CH=CH); 6,23 (N-CH₂-CH=CH) ; 6,46 (N-CH₂-CH=CH) ; 3,56 (Ph-CH₂-N) ; 4,44 4,54 (AB, 2H) et 4,20 4,27 (AB, 2H) : =C-N-CH₂-Cq et Ph-O-CH₂-Cq ; 6,80, 7,14 à 7,24 O-Ph ; 6,80 (2H) 7,38 (1H) 3H imidazole Ha, Hb et Hc ; 6,84 et 7,56 H2 et H4 ; 7,25 à 7,29 H du phényle.

### Compositions pharmaceutiques

On a préparé des composés renfermant

| | |
|---|---|
| Produit de l'exemple 1 | 50 mg |
| Excipient q.s.p. | 1 g |

Détail de l'excipient: amidon, talc, stéarate de magnésium.

### Activité biologique

### 1) Activité antifongique des composés selon l'invention.

On utilise des souris femelles pesant de 18 à 22 g. On leur administre dans la veine de la queue une quantité de Candida Albicans 44858 à raison de 10⁶CFU par souris (CFU : unité formant des colonies). On sépare les souris en 5 lots de 5 souris et on les traite de la façon suivante :
Une heure après l'infection
- groupe 1 : les souris sont traitées avec le produit P 25mg/kg par voie orale
- groupe 2 : les souris sont traitées avec le produit P par voie intrapéritonéale à raison de 25mg/kg
- groupe 3 : les souris sont traitées avec le fluconazole (25mg/kg par voie orale).
- groupe 4 : les souris sont traitées avec le fluconazole (25mg/kg par voie intrapéritonéale).
- groupe 5 : les souris ne reçoivent aucun traitement antifongique.
Pendant une période de 22 jours, on compte les souris mortes.

### 2) Concentration minimale inhibitrice (CMI)

Des cellules de Candida albicans sont préparées comme indiqué dan Journal of Antimicrobial chemotherapy 38, 579-587, lavées 3 fois avec une solution 0,1 M de phosphate et utilisées immédiatement pour déterminer la concentration minimale inhibitrice (CMI).

Les CMI sont déterminés par la modification d'une plaque microtitré selon la méthode standard du Comité National des standards cliniques de laboratoire.
On utilise comme milieu RPMI-1640, et de la L-glutamine tamponnée à pH7 avec une solution 0,15 M de MOPS (acide 3-[N-morpholino]propane sulfonique). On ajoute les cellules de Candida albicans (1,5 X 10³ cellules/ml) dans les puits d'une plaque de 96 puits contenant RPMI-1640 et les dilutions d'agents antifongiques. On fait la lecture des résultats 48 heures après incubation à 35°C et on détermine la CMI ou concentration minimale inhibitrice qui inhibe la croissance des cellules du Candida albicans.

### 3) Concentration minimale fongicide

Après la lecture à 48 heures des CMI, on secoue les plaques et retire 10µL d'aliquote des puits que l'on place sur des disques rectangulaires contenant du dextrose agar. Les plaques sont incubées pendant 48 heures à 35°C ; La concentration minimale fongicide et la concentration de l'agent antifongique à laquelle le nombre d'unité formant des colonies est zéro.

### Conclusion

Les composés selon l'nvention décrits aux exemples 1 à 5 présentent une activité à < 100µg / ml dans le test CMI.

## Revendications

1. Les composés de formule (I) 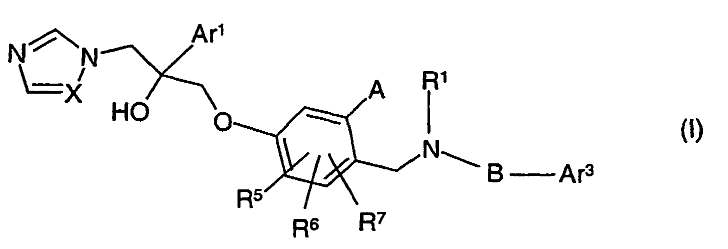 dans laquelle
- X est un atome d'azote ou un groupement CH,
- Ar¹ représente un aryle carbocyclique ou hétérocyclique non substitué ou substitué par un ou plusieurs radicaux R², R³ ou R⁴
- A représente un atome d'hydrogène ou représente un groupement CH₂ formant avec R1 une liaison carbone-carbone cyclique, afin d'obtenir un cycle à 6 chainons accolé au phényle,
- Ar³ représente un aryle carbocyclique ou hétérocyclique non substitué ou substitué par un ou plusieurs radicaux R⁸, R⁹ ou R¹⁰
- B représente un radical (C₁-C₄)-alkylène-CH=CH- ou un radical (C₁-C₄)-alkylène-cyclopropylène, les dits radicaux cyclopropylene ou -CH=CH- étant non substitués ou substitués par un radical R² et/ou R³,
- R¹ représente un atome d'hydrogène, un groupement -SO₃H, un radical (C₁-C₆)-alkyle non substitué ou substitué par un radical tel que défini pour R², ou représente un groupement CH₂ formant avec A une liaison carbone-carbone cyclique, afin d'obtenir un cycle à 6 chainons accolé au phényle,
- R², R³, R⁴, R⁵, R⁶ R⁷, R⁸, R⁹ ou R¹⁰ identiques ou différents, représentent fluor, chlore, brome, cyano, mono- bi- ou trihalogeno (C₁-C₈) alkyle, mono- bi- ou trihalogeno(C₁-C₈)-alkyloxy, hydroxy, nitro, carboxyle, formyle, -SO₃H, - OSO₃H, (R¹¹O)₂P(O)-, (R¹¹O)₂P(O)-O-, amino, (C₁-C₈)-alkylamino, di((C₁-C₈)alkyl)amino, (C₅-C₁₄)-aryl-(C₁-C₆)-alkylènamino ou (C₅-C₁₄)-arylamino, (C₁-C₈)-alkyle, (C₅-C₁₄)-aryle, un hétérocycle éventuellement substitué par oxo, (C₅-C₁₄)-aryl- (C₁-C₆)alkyle, amino- (C₁-C₆)-alkyle, (C₁-C₈)-alkylamino- (C₁-C₆)-alkyle, di-((C₁-C₈)alkyl)amino-(C₁-C₆)-alkyle, hydroxy-(C₁-C₆)alkyle, (C₁-C₆)-alkyloxy-(C₁-C₆)-alkyle, (C₁-C₈)-alkyloxy éventuellement interrompu par un ou plusieurs atomes d'oxygène, (C₅-C₁₄)-aryl-(C₁-C₆)-alkylènoxy, (C₅-C₁₄)-aryloxy, hydroxy-(C₁-C₆)alkylènoxy, (C₁-C₆) -alkyloxy- (C₁-C₆)alkylènoxy, amino- (C₁-C₆)-alkylènoxy, (C₁-C₆)-alkylamino- (C₁-C₆)-alkylènoxy, di ((C₁-C₈)-alkyl)amino-(C₁-C₆)-alkylènoxy, méthylènedioxy, (C₁-C₆)-alkyloxycarbonyle, (C₁-C₆)-alkylcarbonyle, (C₅-C₁₄)aryl-(C₁-C₆)-alkylènecarbonyl, (C₅-C₁₄)-aryl-carbonyle, (C₁-C₆)-alkylaminocarbonyle, (C₁-C₆)alkanoylamino, (C₁-C₆)-alkylsulfonylamino, (C₅-C₁₄)-arylsulfonylamino, (C₅-C₁₄)-aryl-(C₁-C₆)-alkylènesulfonylamino, (C₁-C₆)-alkylamino-sulfonyle, (C₅-C₁₄)-aryl-(C₁-C₆)-alkylènaminosulfonyl, (C₁-C₆)-alkylsulfonyl, (C₅-C₁₄)-aryl-(C₁-C₈)-alkylènesulfonyle ou (C₅-C₁₄)-aryl-sulfonyle, les dits radicaux alkyles, aryles ou hétérocycles étant eux même non substitués ou substitués par un ou plusieurs groupements cités ci-dessus.
- R¹¹ représente hydrogène, (C₁-C₁₀)-alkyl, (C₆-C₁₄)-aryle ou (C₆-C₁₄) -aryl- (C₁-C₆)-alkyle,
sous toutes leurs formes stéréoisomères possibles et leurs mélanges,ainsi que leurs sels d'addition physiologiquement acceptables et leurs prodrogues.

2. les composés de formule (I) tels que définis à la revendication 1 dans laquelle B est un groupement -CH₂-CH=CHou -CH₂-(cyclopropyle)-, les dits groupements étant non substitués ou substitués par un ou plusieurs halogènes ou (C₁-C₄)-alkyle, Ar¹ représente un phényle ainsi que leurs sels d'addition physiologiquement acceptables.

3. Les composés de formule (I) tels que définis à la revendication 1, répondant à la formule (IA) : 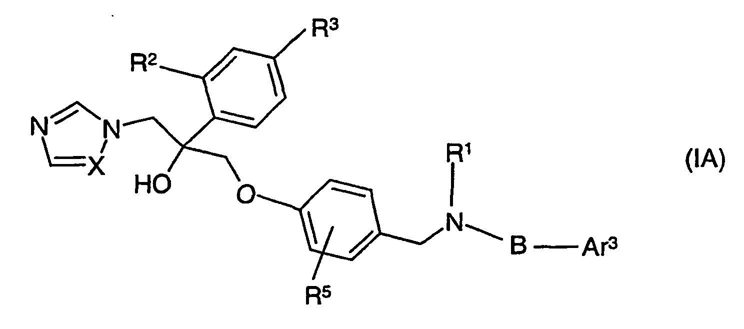 dans laquelle, B, X, Ar³, R⁵ et R¹ sont tels que définis à la revendication 1 ou 2, R² et R³ représentent un atome d'halogène ainsi que leurs sels d'addition physiologiquement acceptables.

4. Les composés de formule (I) tels que définis à la revendication 1 répondant à la formule (Ib) : 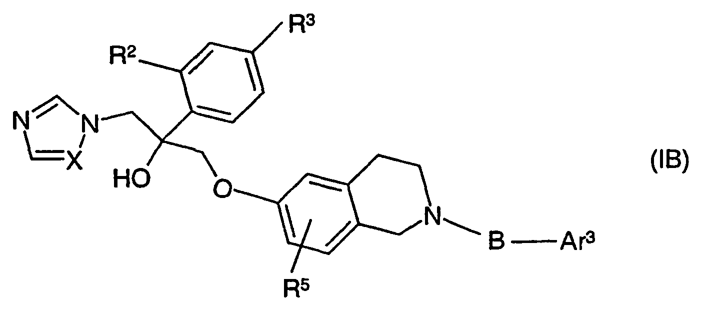 dans laquelle, B, X, Ar³, R⁵ et R¹ sont tels que définis à la revendication 1 ou 2, R² et R³ représentent un atome d'halogène ainsi que leurs sels d'addition physiologiquement acceptables.

5. Les composés de formules (I), (IA) ou (IB) tels que définis aux revendications 1 à 3 **caractérisés en ce que** R₂ et R₃ sont des atomes de fluor ou de chlore, X représente CH ou N et Ar³ représente un groupement phényle non substitué ou substitué par R⁸ tel que défini à la revendication 1, ainsi que leurs sels d'addition physiologiquement acceptables.

6. Les composés de formules (I) ou (IA) tels que définis aux revendications 1 à 4 **caractérisés en ce que** R¹ est un atome d'hydrogène ou un groupement méthyle ou éthyle non substitué ou substitué par un groupement F, OH, NH₂, (C₁-C₆)-alkyloxy, (C₁-C₆)-alkylamino, pyrrolidino, 2-oxopyrrolidino ou di-(C₁-C₆)-alkylamino, ainsi que leurs sels d'addition physiologiquement acceptables.

7. Les composés de formules (I), (IA) ou (IB) tels que définis aux revendications 1 à 4 **caractérisés en ce que** Ar³ est un phényle non susbtitué ou substitué par R⁸ représentant un radical -Cl, -F, CN, -CF₃, -OCF₃, -OH, -NH₂, (C₁-C₆)-alkyloxy, (C₁-C₆)-alkylamino, ou di-(C₁-C₆)-alkylamino ou un hétérocycle choisi parmi :

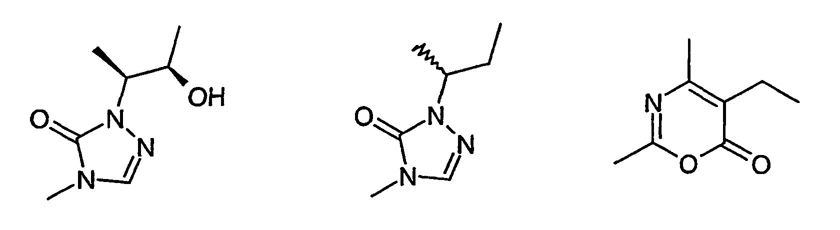

8. Les composés de formules (I), (IA) ou (IB) tels que définis à l'une quelconque des revendications 1 à 7 suivantes :
- alpha-[[[2-[3-(4-chlorophényl)-2(E)-propényl]-1,2,3,4-tétrahydro-6-isoquinoleinyl]oxy]méthyl]-alpha-(2,4-dichlorophényl)-1H-imidazol-1-éthanol
- alpha-(2,4-dichlorophényl)-alpha-[[4-[[[méthyl(3-phényl-2(E)-propényl)]amino]méthyl]phénoxy]méthyl]-1H-imidazol-1-éthanol
- alpha-(2,4-difluorophényl)-alpha-[[4-[[méthyl(3-phényl-2(E)-propényl)amino]méthyl]phénoxy]méthyl]-1H-1,2,4-triazol-1-éthanol
- alpha-[[[2-[3-(4-chlorophényl)-2(E)-propényl]-1,2,3,4-tétrahydro-6-isoquinoleinyl]oxy]méthyl]-alpha-(2,4-difluorophényl)-1H-1,2,4-triazol-1-éthanol
- alpha-(2,4-difluorophényl)-alpha-[[4-[[(2-aminoéthyl)[3-(4-chlorophényl)-2(E)-propényl]amino]méthyl]phénoxy]méthyl]-1H-imidazol-1-éthanol

9. Un procédé de préparation des composés de formule (I) tels que définis à l'une quelconque des revendication 1 à 8 **caractérisé en ce que** l'on soumet un composé de formule (II) 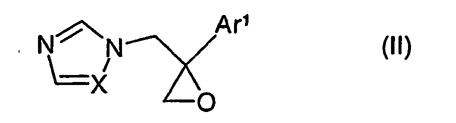 dans laquelle X et Ar¹ sont tels que défini à la revendication 1, à l'action d'un composé de formules (IIIa) ou (IIIb) 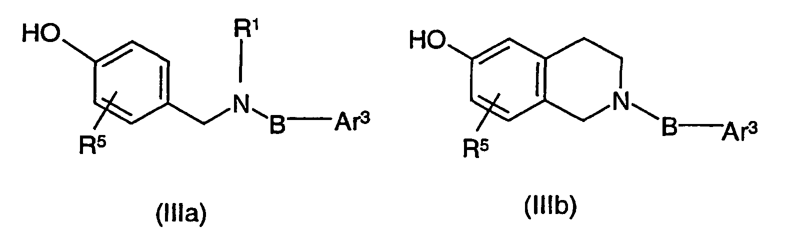 dans laquelle R¹, B, R⁵ et Ar³ sont tels que définis à la revendication 1, en milieu basique, pour obtenir le composé de formule (IA) ou (IB) correspondant tel que défini à la revendication 3 ou 4.

10. Procédé de préparation des composés de formule (I) tels que définis à l'une quelconque des revendication 1 à 7 **caractérisé en ce que** l'on soumet le composé de formule (II) tel que défini à la revendication 8 à l'action d'un aryle de formule (III') HO-C₆H₄-CHO, en présence d'une base, le phényle étant non substitué ou substitué par R⁵ afin d'obtenir un composé de formule (IIA) 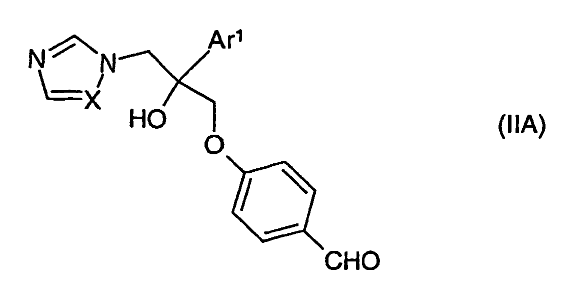 que l'on fait réagir avec une amine de formule R¹-NH₂, R¹ étant tel que défini à la revendication 1 dont les fonctions réactives sont éventuellement protégée, suivi d'une réaction de réduction en présence d'un agent réducteur tel que NaBH₃CN ou BH₃.pyridine, afin d'obtenir un composé de formule (IIB) 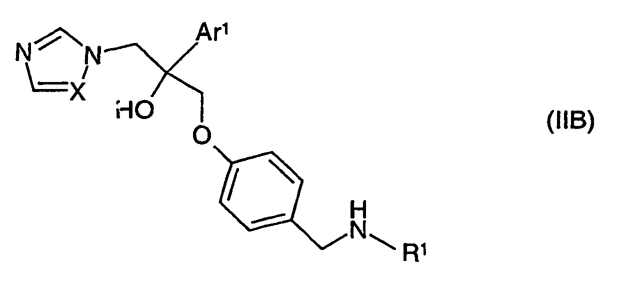 que l'on fait réagir
- soit avec un dérivé de formule
OHC-CH=CH-C₆H₄-R⁸ ou OHC-(Cyclopropyle)-C₆H₄-R⁸
suivi d'une réaction de réduction en présence d'un agent de réduction tel que NaBH₃CN ou BH₃.pyridine
- soit avec un composé de formule :
- AcO-CH₂-CH=CH-C₆H₄-R⁸
en présence d'un dérivé du palladium
afin d'obtenir les composés de formules (IAA)ou(IAB) suivantes :

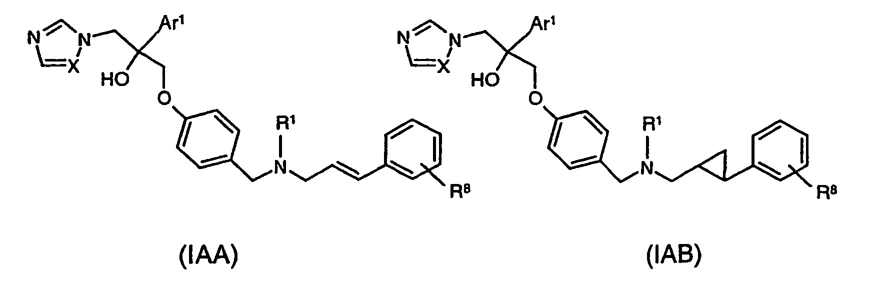

11. A titre de médicament les composés de formule (I) tels que définis à l'une quelconque des revendications 1 à 8 ainsi que leurs sels d'addition pharmaceutiquement acceptables et/ou leurs prodrogues.

12. Les composés de formule (I) tels que définis à l'une quelconque des revendications 1 à 8 ainsi que leurs sels d'addition pharmaceutiquement acceptables et/ou leurs prodrogues pour leur utilisation à titre d'antifongique.

13. Composition pharmaceutique renfermant au moins 1 composé de formule (I) tels que définis à l'une quelconque des revendications 1 à 8 ainsi que leurs sels d'addition pharmaceutiquement acceptables et/ou leurs prodrogues et un véhicule pharmaceutiquement acceptables.

14. A titre de composés intermédiaires, les composés de formules (IIa) et (IIb) tels que définis à la revendication 10.

## Claims

1. Compounds of formula (I) 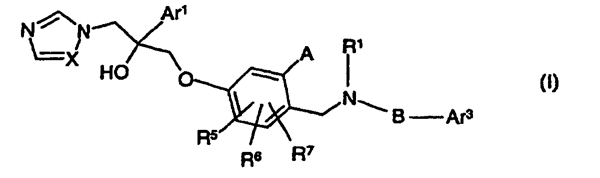 in which
- X is a nitrogen atom or a CH group,
- Ar¹ represents a carbocyclic or heterocyclic aryl that is unsubstituted or substituted with one or more radicals R², R³ or R⁴,
- A represents a hydrogen atom or represents a CH₂ group that forms, with R¹, a cyclic carbon-carbon bond, in order to obtain a 6-membered ring joined to the phenyl,
- Ar³ represents a carbocyclic or heterocyclic aryl that is unsubstituted or substituted with one or more radicals R⁸, R⁹ or R¹⁰,
- B represents a (C₁-C₄)alkylene-CH=CH- radical or a (C₁-C₄)alkylene-cyclopropylene radical, said cyclopropylene or -CH=CH- radicals being unsubstituted or substituted with a radical R² and/or R³,
- R¹ represents a hydrogen atom, an -SO₃H group, or a (C₁-C₆)alkyl radical that is unsubstituted or substituted with a radical as defined for R², or represents a CH₂ group that forms, with A, a cyclic carbon-carbon bond, in order to obtain a 6-membered ring joined to the phenyl,
- R², R³, R⁴, R⁵, R⁶, R⁷, R⁸, R⁹ or R¹⁰, which may be identical or different, represent fluorine, chlorine, bromine, cyano, mono-, bi- or trihalo-(C₁-C₈)alkyl, mono-, bi- or trihalo(C₁-C₈)alkyloxy, hydroxyl, nitro, carboxyl, formyl, -SO₃H, -OSO₃H, (R¹¹O)₂P(O)-, (R¹¹O)₂P(O)-O-, amino, (C₁-C₈)alkylamino, di ((C₁-C₈)alkyl)amino, (C₅-C₁₄)aryl (C₁-C₆)-alkyleneamino or (C₅-C₁₄)arylamino, (C₁-C₈)alkyl, (C₅-C₁₄)aryl, a heterocycle optionally substituted with oxo, (C₅-C₁₄)aryl(C₁-C₆)alkyl, amino(C₁-C₆)alkyl, (C₁-C₈)alkylamino (C₁-C₆)alkyl, di((C₁-C₈)alkyl)amino(C₁-C₆)alkyl, hydroxy(C₁-C₆)alkyl, (C₁-C₆) alkyloxy(C₁-C₆) alkyl, (C₁-C₈)alkyloxy optionally interrupted with one or more oxygen atoms, (C₅-C₁₄)aryl(C₁-C₆)alkyleneoxy, (C₅-C₁₄)aryloxy, hydroxy(C₁-C₆)alkyleneoxy, (C₁-C₆)alkyloxy(C₁-C₆)alkyleneoxy, amino (C₁-C₆)alkyleneoxy, (C₁-C₆)-alkylamino(C₁-C₆)alkyleneoxy, di ((C₁-C₈)alkyl) amino-(C₁-C₆)alkyleneoxy, methylenedioxy, (C₁-C₆)alkyloxycarbonyl, (C₁-C₆) alkylcarbonyl, (C₅-C₁₄)aryl(C₁-C₆)-alkylenecarbonyl, (C₅-C₁₄)arylcarbonyl, (C₁-C₆)-alkylaminocarbonyl, (C₁-C₆)alkanoylamino, (C₁-C₆)-alkylsulphonylamino, (C₅-C₁₄)arylsulphonylamino, (C₅-C₁₄)aryl(C₁-C₆)alkylenesulphonylamino, (C₁-C₆)-alkylaminosulphonyl, (C₅-C₁₄)aryl (C₁-C₆)alkylene-aminosulphonyl, (C₁-C₆)alkylsulphonyl, (C₅-C₁₄)aryl-(C₁-C₈)alkylenesulphonyl or (C₅-C₁₄) arylsulphonyl, said alkyl, aryl or heterocycle radicals being themselves unsubstituted or substituted with one or more groups mentioned above,
- R¹¹ represents hydrogen, (C₁-C₁₀)alkyl, (C₆-C₁₄)aryl or (C₆-C₁₄)aryl(C₁-C₆)alkyl,
in any of their possible stereoisomeric forms and their mixtures, and also their physiologically acceptable addition salts and their prodrugs.

2. Compounds of formula (I) as defined in Claim 1, in which B is a -CH₂-CH=CH- or -CH₂-(cyclopropyl)- group, said groups being unsubstituted or substituted with one or more halogens or (C₁-C₄)alkyl and Ar¹ represents a phenyl, and also their physiologically acceptable addition salts.

3. Compounds of formula (I) as defined in Claim 1, corresponding to formula (IA): 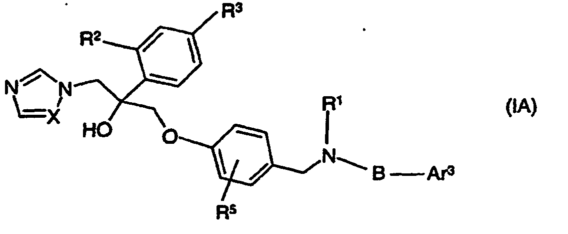 in which B, X, Ar³, R⁵ and R¹ are as defined in Claim 1 or 2, and R² and R³ represent a halogen atom, and also their physiologically acceptable addition salts.

4. Compounds of formula (I) as defined in Claim 1, corresponding to formula (IB): 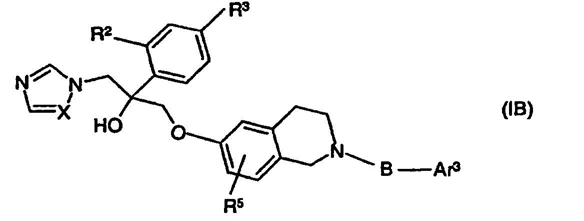 in which B, X, Ar³, R⁵ and R¹ are as defined in Claim 1 or 2, and R² and R³ represent a halogen atom, and also their physiologically acceptable addition salts.

5. Compounds of formulae (I), (IA) or (IB) as defined in Claims 1 to 3, **characterized in that** R² and R³ are fluorine or chlorine atoms, X represents CH or N and Ar³ represents a phenyl group that is unsubstituted or substituted with R⁸ as defined in Claim 1, and also their physiologically acceptable addition salts.

6. Compounds of formulae (I) or (IA) as defined in Claims 1 to 4, **characterized in that** R¹ is a hydrogen atom or a methyl or ethyl group that is unsubstituted or substituted with an F, OH, NH₂, (C₁-C₆)alkyloxy, (C₁-C₆)alkylamino, pyrrolidino, 2-oxopyrrolidino or di(C₁-C₆)alkylamino group, and also their physiologically acceptable addition salts.

7. Compounds of formulae (I), (IA) or (IB) as defined in Claims 1 to 4, **characterized in that** Ar³ is a phenyl that is unsubstituted or substituted with R⁸ representing a -Cl, -F, CN, -CF₃, -OCF₃, -OH, -NH₂, (C₁-C₆) alkyloxy, (C₁-C₆)alkylamino or di(C₁-C₆)alkylamino radical, or a heterocycle chosen from:

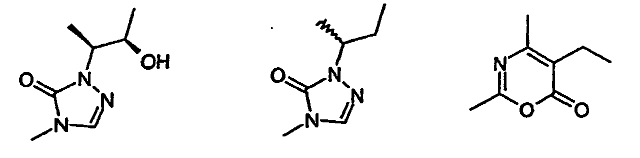

8. Compounds of formulae (I), (IA) or (IB) as defined in any one of Claims 1 to 7, below:
- alpha-[[[2-[3-(4-chlorophenyl)-2(E)-propenyl]-1,2,3,4-tetrahydro-6-isoquinolinyl]oxy]methyl]-alpha-(2,4-dichlorophenyl)-1H-imidazole-1-ethanol
- alpha-(2,4-dichlorophenyl)-alpha-[[4-[[[methyl-(3-phenyl-2(E)-propenyl)]amino]methyl]phenoxy]methyl]-1H-imidazole-1-ethanol
- alpha-(2,4-difluorophenyl)-alpha-[[4-[[methyl-(3-phenyl-2(E)-propenyl)amino]methyl]phenoxy]methyl]-1H-1,2,4-triazole-1-ethanol
- alpha-[[[2-[3-(4-chlorophenyl)-2(E)-propenyl]-1,2,3,4-tetrahydro-6-isoquinolinyl]oxy]methyl]-alpha-(2,4-difluorophenyl)-1H-1,2,4-triazole-1-ethanol
- alpha-(2,4-difluorophenyl)-alpha-[[4-[[(2-aminoethyl) [3-(4-chlorophenyl)-2(E)-propenyl]amino]methyl]-phenoxy]methyl]-1H-imidazole-1-ethanol.

9. Method for preparing the compounds of formula (I) as defined in any one of Claims 1 to 8, **characterized in that** a compound of formula (II) 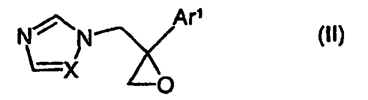 in which X and Ar¹ are as defined in Claim 1, is subjected to the action of a compound of formula (IIIa) or (IIIb) 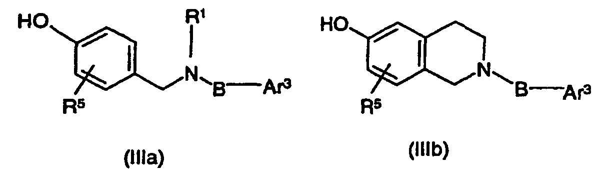 in which R¹, B, R⁵ and Ar³ are as defined in Claim 1, in basic medium, so as to obtain the corresponding compound of formula (IA) or (IB) as defined in Claim 3 or 4.

10. Method for preparing the compounds of formula (I) as defined in any one of Claims 1 to 7, **characterized in that** the compound of formula (II) as defined in Claim 8 is subjected to the action of an aryl of formula (III') HO-C₆H₄-CHO, in the presence of a base, the phenyl being unsubstituted or substituted with R⁵, in order to obtain a compound of formula (IIA) 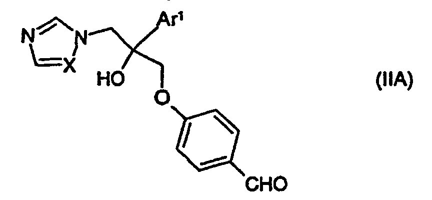 which is reacted with an amine of formula R¹-NH₂, R¹ being as defined in Claim 1, the reactive functions of which are optionally protected, followed by a reduction reaction in the presence of a reducing agent such as NaBH₃CN or BH₃.pyridine, in order to obtain a compound of formula (IIB) 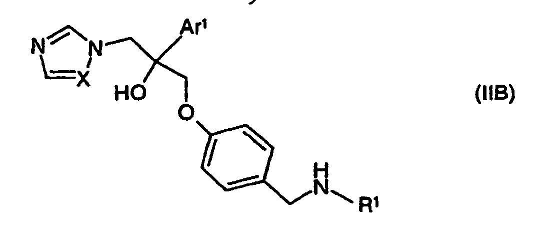 which is reacted
- either with a derivative of formula
OHC-CH=CH-C₆H₄-R⁸ or OHC-(cyclopropyl)-C₆H₄-R⁸
followed by a reduction reaction in the presence of a reducing agent such as NaBH₃CN or BH₃.pyridine
- or with a compound of formula:
- AcO-CH₂-CH=CH-C₆H₄-R⁸
in the presence of a palladium derivative
in order to obtain the compounds of formulae (IAA) or (IAB) below:

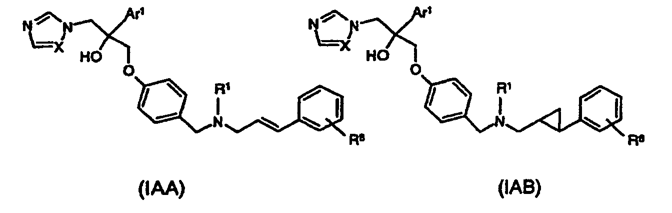

11. Compounds of formula (I) as defined in any one of Claims 1 to 8, and also their pharmaceutically acceptable addition salts and/or their prodrugs, as a medicinal product.

12. Compounds of formula (I) as defined in any one of Claims 1 to 8, and also their pharmaceutically acceptable addition salts and/or their prodrugs, for their use as an antifungal agent.

13. Pharmaceutical composition containing at least one compound of formula (I) as defined in any one of Claims 1 to 8, and also their pharmaceutically acceptable addition salts and/or their prodrugs, and a pharmaceutically acceptable vehicle.

14. Compounds of formulae (IIA) and (IIB) as defined in Claim 10, as intermediate compounds.

## Patentansprüche

1. Verbindungen der Formel (I) 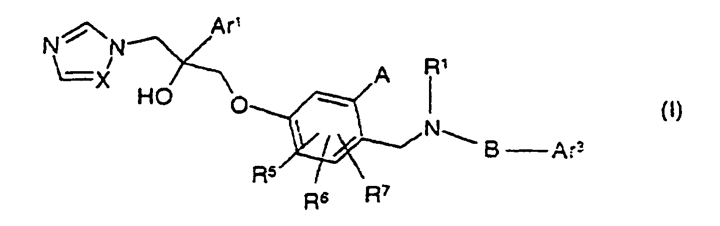 in der
- X ein Stickstoffatom oder eine Gruppe CH ist,
- Ar¹ ein carbocyclisches oder heterocyclisches Aryl darstellt, nicht substituiert oder substituiert durch einen oder mehrere Reste R², R³ oder R⁴,
- A ein Wasserstoffatom oder eine Gruppe CH₂ darstellt, die zusammen mit R¹ eine cyclische Kohlenstoff-Kohlenstoff-Bindung bildet, um einen Ring mit 6 Kettengliedern zu erhalten, angefügt an Phenyl,
- Ar³ ein carbocyclisches oder heterocyclisches Aryl darstellt, nicht substituiert oder substituiert durch einen oder mehrere Reste R⁸, R⁹ oder R¹⁰,
- B einen Rest (C₁-C₄)-Alkylen-CH=CH- oder einen Rest (C₁-C₄)-Alkylen-cyclopropylen darstellt, wobei die genannten Reste Cyclopropylen oder -CH=CH- nicht substituiert oder substituiert sind durch einen Rest R² und/oder R³,
- R¹ ein Wasserstoffatom, eine Gruppe -SO₃H, einen Rest (C₁-C₆)-Alkyl, nicht substituiert oder substituiert durch einen wie für R² definierten Rest, oder eine Gruppe CH₂ darstellt, die zusammen mit A eine cyclische Kohlenstoff-Kohlenstoff-Bindung bildet, um einen Ring mit 6 Kettengliedern zu erhalten, angefügt an Phenyl,
- R², R³, R⁴, R⁵, R⁶, R⁷, R⁸, R⁹ oder R¹⁰, gleich oder verschieden, Fluor, Chlor, Brom, Cyano, Mono-, Di- oder Trihalogen-(C₁-C₈)-alkyl, Mono-, Di- oder Trihalogen-(C₁-C₈)-alkyloxy, Hydroxy, Nitro, Carboxyl, Formyl, -SO₃H, -OSO₃H, (R¹¹O)₂P(O)-, (R¹¹O)₂P(O)-O-, Amino, (C₁-C₈)-Alkylamino, Di-[(C₁-C₈)-alkyl]-amino, (C₅-C₁₄)-Aryl-(C₁-C₆)-alkylenamino oder (C₅-C₁₄)-Arylamino, (C₁-C₈)-Alkyl, (C₅-C₁₄)-Aryl, einen Heterocyclus, gegebenenfalls substituiert durch Oxo, (C₅-C₁₄)-Aryl-(C₁-C₆)-alkyl, Amino-(C₁-C₆)-alkyl, (C₁-C₈)-Alkylamino-(C₁-C₆)-alkyl, Di- [(C₁-C₈)-alkyl] -amino- (C₁-C₆) -alkyl, Hydroxy- (C₁-C₆) -alkyl, (C₁-C₆)-Alkyloxy-(C₁-C₆)-alkyl, (C₁-C₈)-Alkyloxy, gegebenenfalls unterbrochen durch ein oder mehrere Sauerstoffatome, (C₅-C₁₄)-Aryl-(C₁-C₆)-alkylenoxy, (C₅-C₁₄)-Aryloxy, Hydroxy- (C₁-C₆)-alkylenoxy, (C₁-C₆) -Alkyloxy- (C₁-C₆)-alkylenoxy, Amino-(C₁-C₆)-alkylenoxy, (C₁-C₆)-Alkylamino-(C₁-C₆)-alkylenoxy, Di- [(C₁-C₈)-alkyl]-amino-(C₁-C₆)-alkylenoxy, Methylendioxy, (C₁-C₆)-Alkyloxycarbonyl, (C₁-C₆)-Alkylcarbonyl, (C₅-C₁₄)-Aryl- (C₁-C₆)-alkylencarbonyl, (C₅-C₁₄)-Arylcarbonyl, (C₁-C₆)-Alkylaminocarbonyl, (C₁-C₆) -Alkanoylamino, (C₁-C₆)-Alkylsulfonylamino, (C₅-C₁₄)-Arylsulfonyl-amino, (C₅-C₁₄)-Aryl-(C₁-C₆)-alkylensulfonylamino, (C₁-C₆)-Alkylaminosulfonyl, (C₅-C₁₄)-Aryl-(C₁-C₆)-alkylenaminosulfonyl, (C₁-C₆)-Alkylsulfonyl, (C₅-C₁₄)-Aryl-(C₁-C₈)-alkylensulfonyl oder (C₅-C₁₄)-Arylsulfonyl darstellen, wobei die genannten Reste Alkyl, Aryl oder die heterocyclischen Reste selbst nicht substituiert oder substituiert sind durch eine oder mehrere der oben genanten Gruppen,
- R¹¹ Wasserstoff, (C₁-C₁₀)-Alkyl, (C₆-C₁₄)-Aryl oder (C₆-C₁₄)-Aryl-(C₁-C₆)-alkyl darstellt,
in allen ihren möglichen stereoisomeren Formen und ihren Mischungen, sowie ihre physiologisch akzeptablen Additionssalze und ihre Prodrugs.

2. Verbindungen der Formel (I) wie in Anspruch 1 definiert, worin B eine Gruppe -CH₂-CH=CH- oder -CH₂-(cyclopropyl)- darstellt, wobei die genannten Gruppen nicht substituiert oder substituiert sind durch ein oder mehrere Halogene oder (C₁-C₄)-Alkyl, und Ar¹ ein Phenyl bedeutet, sowie ihre physiologisch akzeptablen Additionssalze.

3. Verbindungen der Formel (I) wie in Anspruch 1 definiert, entsprechend der Formel (IA) 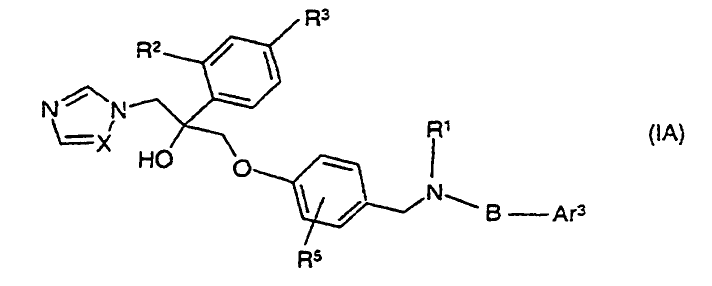 in der
B, X, Ar³, R⁵ und R¹ wie in Anspruch 1 oder 2 definiert sind, R² und R³ ein Halogenatom darstellen, sowie ihre physiologisch akzeptablen Additionssalze.

4. Verbindungen der Formel (I) wie in Anspruch 1 definiert, entsprechend der Formel (IB) 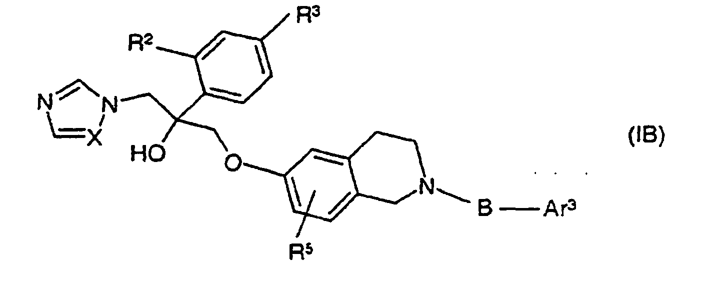 in der
B, X, Ar³, R⁵ und R¹ wie in Anspruch 1 oder 2 definiert sind, R² und R³ ein Halogenatom darstellen, sowie ihre physiologisch akzeptablen Additionssalze.

5. Verbindungen der Formeln (I), (IA) oder (IB) wie in den Ansprüchen 1 bis 3 definiert, **dadurch gekennzeichnet, daß** R₂ und R₃ Atome von Fluor oder Chlor sind, X CH oder N darstellt und Ar³ eine Gruppe Phenyl bedeutet, nicht substituiert oder substituiert durch R⁸ wie in Anspruch 1 definiert, sowie ihre physiologisch akzeptablen Additionssalze.

6. Verbindungen der Formeln (I) oder (IA) wie in den Ansprüchen 1 bis 4 definiert, **dadurch gekennzeichnet, daß** R¹ ein Wasserstoffatom oder eine Gruppe Methyl oder Ethyl ist, nicht substituiert oder substituiert durch eine Gruppe F, OH, NH₂, (C₁-C₆)-Alkyloxy, (C₁-C₆)-Alkylamino, Pyrrolidino, 2-Oxypyrrolidino oder Di-(C₁-C₆)-alkylamino, sowie ihre physiologisch akzeptablen Additionssalze.

7. Verbindungen der Formeln (I), (IA) oder (IB) wie in den Ansprüchen 1 bis 4 definiert, **dadurch gekennzeichnet, daß** Ar³ ein Phenyl ist, nicht substituiert oder substituiert durch R⁸, das einen Rest -Cl, -F, CN, -CF₃, -OCF₃, -OH, -NH₂, (C₁-C₆)-Alkyloxy, (C₁-C₆)-Alkylamino oder Di-(C₁-C₆)-alkylamino darstellt, oder einen Heterocyclus bedeutet, ausgewählt unter:

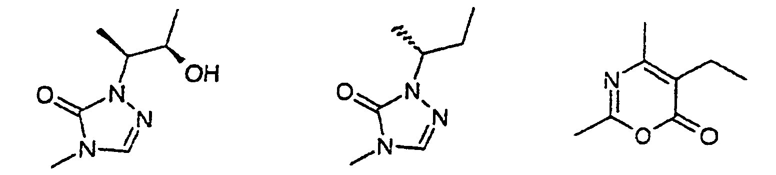

8. Verbindungen der Formeln (I), (IA) oder (IB) wie in irgendeinem der Ansprüche 1 bis 7 definiert, die folgen:
- alpha-{{[2-[3-(4-Chlorphenyl)-2(E)-propenyl]-1,2,3,4-tetrahydro-6-isochinolinyl]-oxy}-methyl}-alpha-(2,4-dichlorphenyl)-1H-imidazol-1-ethanol
- alpha-(2,4-Dichlorphenyl)-alpha-{{4-{[[methyl-(3-phenyl-2(E)-propenyl)]-amino]-methyl}-phenoxy}-methyl}-1H-imidazol-1-ethanol
- alpha-(2,4-Difluorphenyl)-alpha-{{4-[[methyl-(3-phenyl-2(E)-propenyl)-amino]-methyl]-phenoxy}-methyl}-1H-1,2,4-triazol-1-ethanol
- alpha-{{[2-[3-(4-Chlorphenyl)-2(E)-propenyl]-1,2,3,4-tetrahydro-6-isochinolinyl]-oxy}-methyl}-alpha-(2,4-difluorphenyl)-1H-1,2,4-triazol-1-ethanol
- alpha-(2,4-Difluorphenyl)-alpha-{{4-[[(2-aminoethyl)-[3-(4-chlorphenyl)-2(E)-propenyl]-amino]-methyl]-phenoxy}-methyl}-1H-imidazol-1-ethanol.

9. Verfahren zur Herstellung der Verbindungen der Formel (I) wie in irgendeinem der Ansprüche 1 bis 8 definiert, **dadurch gekennzeichnet, daß** man eine Verbindung der Formel (II) 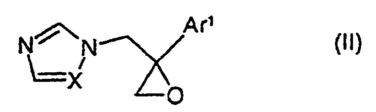 in der X und Ar¹ wie in Anspruch 1 definiert sind, der Einwirkung einer Verbindung der Formel (IIIa) oder (IIIb) 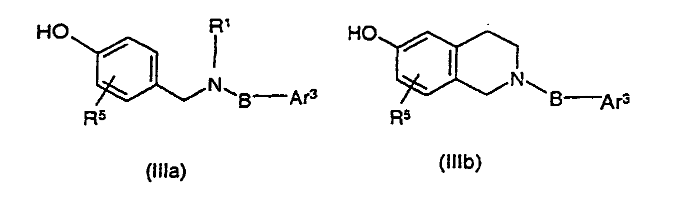 worin R¹, B, R⁵ und Ar³ wie in Anspruch 1 definiert sind, im basischen Medium unterzieht, um die entsprechende Verbindung der Formel (IA) oder (IB) zu erhalten, wie in Anspruch 3 oder 4 definiert.

10. Verfahren zur Herstellung der Verbindungen der Formel (I) wie in irgendeinem der Ansprüche 1 bis 7 definiert, **dadurch gekennzeichnet, daß** man die Verbindung der Formel (II), wie in Anspruch 8 definiert, der Einwirkung eines Aryls der Formel (III') HO-C₆H₄-CHO in Anwesenheit einer Base unterzieht, wobei das Phenyl nicht substituiert oder substituiert ist durch R⁵, um eine Verbindung der Formel (IIA) 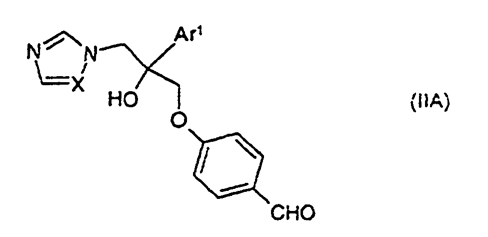 zu erhalten, die man mit einem Amin der Formel R¹-NH₂, worin R¹ wie in Anspruch 1 definiert ist, dessen reaktive Funktionen gegebenenfalls geschützt sind, zur Reaktion bringt, gefolgt von einer Reduktionsreaktion in Anwesenheit eines Reduktionsmittels wie NaBH₃CN oder BH₃.pyridin, um eine Verbindung der Formel (IIB) 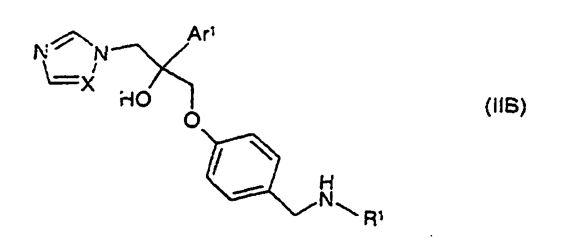 zu erhalten,
die man zur Reaktion bringt
- entweder mit einem Derivat der Formel
OHC-CH=CH-C₆H₄-R⁸ oder OHC-(Cyclopropyl)-C₆H₄-R⁸, gefolgt von einer Reduktionsreaktion in Anwesenheit eines Reduktionsmittels wie NaBH₃CN oder BH₃.pyridin,
- oder mit einer Verbindung der Formel
AcO-CH₂-CH=CH-C₆H₄-R⁸ in Anwesenheit eines Palladiumderivates, um die Verbindungen der Formeln (IAA) oder (IAB) 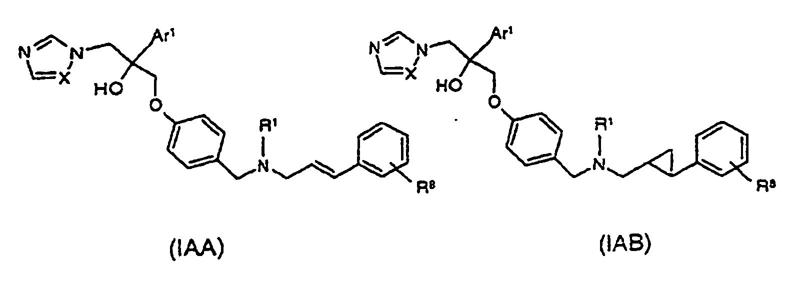 zu erhalten.

11. Als Arzneimittel die Verbindungen der Formel (I) wie in irgendeinem der Ansprüche 1 bis 8 definiert sowie ihre pharmazeutisch akzeptablen Additionssalze und/oder ihre Prodrugs.

12. Verbindungen der Formel (I) wie in irgendeinem der Ansprüche 1 bis 8 definiert sowie ihre pharmazeutisch akzeptablen Additionssalze und/oder ihre Prodrugs für ihre Verwendung als fungizide Mittel.

13. Pharmazeutische Zusammensetzung, umfassend mindestens eine Verbindung der Formel (I) wie in irgendeinem der Ansprüche 1 bis 8 definiert sowie ihre pharmazeutisch akzeptablen Additionssalze und/oder ihre Prodrugs und einen pharmazeutisch akzeptablen Trägerstoff.

14. Als Zwischenverbindungen die Verbindungen der Formeln (IIa) und (IIb) wie in Anspruch 10 definiert.
